# EUROPEAN PATENT APPLICATION

(11) **EP 1 502 952 A2**
(43) Date of publication of application: **02.02.2005**
(21) Application number: 04026492.1
(22) Date of filing: 16.03.1987
(51) Int. Cl.: C12N 15/80, C12N 15/52

(54) **Process for the production of protein products in Aspergillus oryzae and a promoter for use in Aspergillus**

(30) Priority: 17.03.1986 DK 122686
(62) Divisional of application: 92104421.0
(71) Applicant: Novozymes A/S, 2880 Bagsvaerd (DK)
(72) Inventor: Boel, Esper, 2840 Holte (DK); Christensen, Tove, 2800 Lyngby (DK); Woldike, Helle Fabricius, 3540 Lynge (DK)
(74) Representative: Bendiksen, Henrik

(57) **Abstract**

A process for expression of a protein product in Aspergillus oryzae is disclosed. The process comprises transforming Aspergillus oryzae with a vector system comprising DNA-sequences encoding functions facilitating gene expression, a suitable marker for selection of transformants, and a DNA-sequence encoding the desired protein product. The process enables industrial production of many different polypeptides and proteins in A. oryzae. Examples of such products are chymosin or prochymosin and other rennets, proteases, lipases and amylases. Also disclosed is an effective promoter for expression of a protein in Aspergillus. A preferred promoter is the TAKA-amylase promoter or functional parts thereof.

## Description

### BACKGROUND OF THE INVENTION

The present invention relates to a process for expression of protein products in Aspergillus oryzae, recombinant DNA vectors, a promoter for Aspergillus and transformed fungi.

In the past, numerous processes have been developed for the production of polypeptides or proteins by means of the recombinant DNA technology. The main interest has been concentrated on bacteria and yeast, e.g. E. coli, Bacillus subtilis and Saccharomyces cerevisiae being well characterized species as regards for instance expression and selection systems.

Besides the above mentioned microorganisms, filamentous fungi, such as Aspergillus niger, are attractive candidates as host microorganisms for recombinant DNA vectors being well-characterized and widely used microorganisms for the commercial production of enzymes. Efforts have especially been concentrated on the development of transformation systems by which a selection marker permitting selection of transformants from the untransformed host microorganisms is used.

In the last few years different selection markers for the transformation of Aspergillus nidulans have been described and procedures have recently been developed for integrative transformation of the filamentous fungus Aspergillus nidulans for the purpose of investigation of the genetic and molecular processes controlling fungal cell differentiation.

Transformation of A. nidulans has been demonstrated by using plasmids containing the Neurospora crassa pyr-4 gene (Ballance, D.J. et al., Biochem.Biophys.Res.Commun., 112 (1983) 284-289), the A. nidulans amdS gene (Tilburn, J.G. et al., Gene 26 (1983) 205-221), the A. nidulans trpC gene (Yelton, M.M. et al., Proc.Natl.Acad.Sci. U.S.A., 81 (1984) 1470-1474) and the A. nidulans argB gene (John, M.A. and Peberdy J., Microb.Technol. 6 (1984) 386-389). The transforming DNA was found to be integrated into the host genome at rather low frequencies (typically < 1000 transformants/µg of DNA).

Very recently transformation of Aspergillus niger with the amdS gene of A. nidulans was described (Kelly, J.M. and Hynes, M.J., EMBO Journal 4 (1985), 475-479) and amdS was shown to be a potential selection marker for use in transformation of Aspergillus niger that cannot grow strongly on acetamide as a sole nitrogen source. Transformation of Aspergillus niger using the argB gene of Aspergillus nidulans has also been described recently (Buxton, F. P. et al., Gene 37 (1985), 207-214).

So far no systems have been developed for expression of foreign proteins in the filamentous fungi Aspergillus oryzae mainly due to insufficient knowledge of how to control gene expression in this fungus and due to the lack of suitable selectable genetic markers on cloning vectors.

### BRIEF DESCRIPTION OF THE INVENTION

According to the present invention it has now been shown that the above transformation techniques can be used to obtain a high level of expression of heterologous proteins or to enhance the production of homologous proteins in Aspergillus oryzae.

As used herein the expression "heterologous proteins" means proteins not produced by A. oryzae whereas "homologous proteins" means proteins produced by A. oryzae itself.

More specifically it has been shown that selection for A. oryzae strains transformed with DNA encoding a desired protein product is possible by use of the marker genes used for transformation of A. niger and A. nidulans. Due to the phylogenetic distance between these latter fungi and A. oryzae (Raper, K.B. and Fennell, D.I. (1.965) The Genus Aspergillus) this was by no means to be foreseen.

According to a first aspect of the present invention there is provided a process for expression of a protein product in Aspergillus oryzae comprising the steps of:
(a) providing a recombinant DNA cloning vector system capable of integration into the genome of an Aspergillus oryzae host in one or more copies and comprising: DNA-sequences encoding functions facilitating gene expression; a suitable marker for selection of transformants; and a DNA-sequence encoding the desired protein product;
(b) transforming the Aspergillus oryzae host which does not harbour a functional gene for the chosen selection marker with the recombinant DNA cloning vector system from step a; and
(c) culturing the transformed Aspergillus oryzae host in a suitable culture medium.

According to a second aspect of the present invention there is provided a highly effective promoter for expression of a protein product in Aspergillus, especially in Aspergillus oryzae and Aspergillus niger, which promoter is characterized as being the TAKA-amylase promoter or functional parts thereof optionally preceded by upstream activating sequences.

According to a third aspect of the present invention there is provided a method for production of a protein product in Aspergillus oryzae by which method an Aspergillus oryzae strain being transformed with a recombinant DNA cloning vector system as described above is cultured in a suitable culture medium and the product is recovered from the culture medium.

### BRIEF DESCRIPTION OF THE DRAWINGS:

The present invention is further illustrated by reference to the accompanying drawings in which:
Fig. 1 shows the DNA-sequence of the TAKA-amylase promoter and upstream promoter regions, the preregion and the 5'part of the structural gene for the TAKA-amylase.
Fig. 2 shows an endonuclease restriction map of plasmid pTAKA17,
Fig. 3 illustrates the construction of plasmid p285'proC,
Fig. 4a and b shows the DNA sequence of prepro
Rhizomucor miehei aspartic proteinase cDNA together with the deduced amino acid sequence given by three-letter abbreviations,
Fig. 5 illustrates the construction of plasmid pRMP,
Fig. 6 shows the endonuclease restriction map of plasmid pCAMG91
Fig. 7a illustrates the construction of plasmid pICAMG/Term,
Fig. 7b illustrates the construction of plasmid p686
Fig. 8 illustrates the construction of plasmid pRMPAMGTerm,
Fig. 9a illustrates the construction of plasmid pB408.3,
Fig. 9b illustrates the construction of plasmid p778,
Fig. 10 illustrates the construction of plasmid p719,
Fig. 11 illustrates the construction of plasmid p777,
Fig. 12 shows the sequence of prepro Rhizomucor miehei lipase cDNA together with the deduced amino acid sequence given by three-letter abbreviations.
Fig. 13a illustrates the construction of plasmid plasmid pB544,
Fig. 13b illustrates the construction of plasmid p787,
Fig. 14 shows the DNA sequence of a synthetic fragment RML5',
Fig. 15a illustrates the construction of plasmid pTOC51 and
Fig. 15b illustrates the construction of plasmid pTOC56.

### DETAILED DESCRIPTION OF THE INVENTION

The transformation technique used was a method adapted from the methods for transformation of A. nidulans (Ballance et al. Biochem.Biophys.Res.Commun., 112 (1983), 284-289; Tilburn et al., Gene 26 (1983), 205-221, Yelton et al. Proc.Natl.Acad.Sci. USA, 81 (1984), 1470-1474) and similar to the method of Buxton et al. (Gene 37 (1985), 207-214) for transformation of A. niger. In the process of the present invention Aspergillus oryzae is transformed with a vector system containing a selection marker which is capable of being incorporated into the genome of the host strain, but which is not harboured in the host strain before the transformation. Transformants can then be selected and isolated from nontransformants on the basis of the incorporated selection marker.

Preferred selection markers are the argB (A. nidulans or A. niger), trpC (A. nidulans), amdS (A. nidulans), or pyr4 (Neurospora crassa) genes, or the DHFR (dihydrofolate reductase or mutants hereof) gene. More preferred selection markers are the argB or the amdS gene. Wild type A. oryzae strains are normally argB⁺ (i.e. the argB gene is functional in A. oryzae). If argB is chosen as the selection marker an argB mutant strain of A. oryzae which has a defect in the gene for this marker must be used as host strain. A. oryzae argB mutants can be prepared as described by F.P. Buxton et al. (Gene 37 (1985), 207-214). An argB mutant is defined as a mutant having a defect in the ornithin transcarbamylase gene. On the other hand the amdS gene may be used as selection marker for the transformation of wild type A. oryzae as the wild type strains do not contain this gene.

DNA-sequences encoding functions facilitating gene expression are typically promoters, transcription terminators and polyadenylation signals.

The promoter, which might be preceded by upstream activating sequences and enhancer sequences as well known in the art, may be any DNA-sequence that might show strong transcriptional activity in Aspergillus oryzae and may be derived from genes encoding both extracellular and intracellular proteins, such as amylases, glucoamylases, proteases, lipases, cellulases and glycolytic enzymes. Suitable promoters may be derived from genes for A. oryzae TAKA amylase, Rhizomucor miehei aspartic proteinase, A. niger glucoamylase, A. niger neutral α-amylase, A. niger acid stable α-amylase, and Rhizomucor miehei lipase. Examples of promoters from genes for glycolytic enzymes are TPI, ADH and PGK.

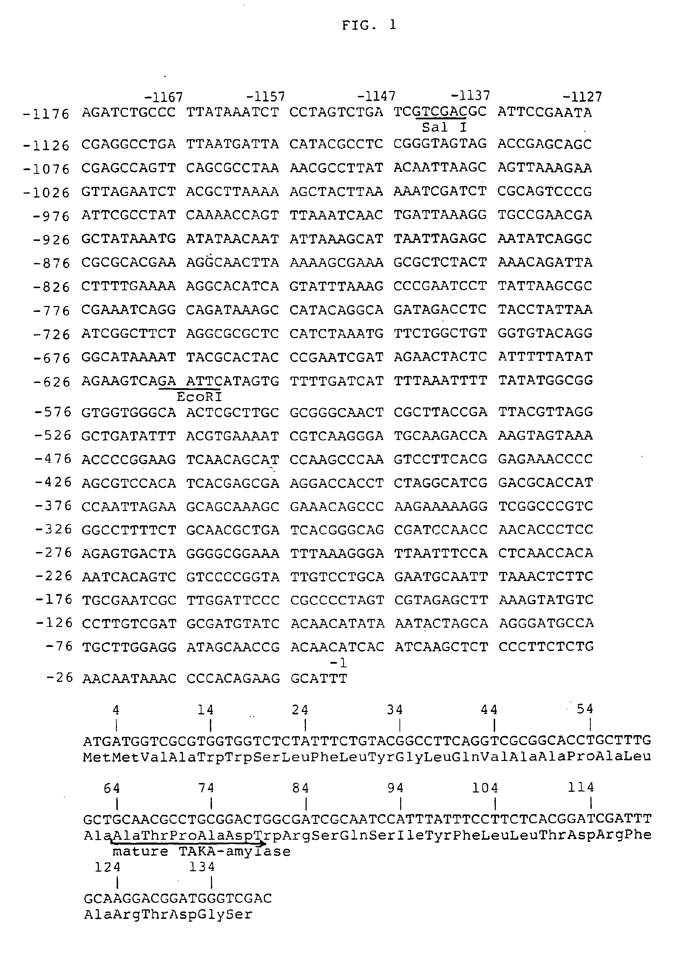

A preferred promoter according to the present invention is the A. oryzae TAKA-amylase promoter. The TAKA amylase is a well known α-amylase (Toda et al., Proc.Japan Acad. 58 Ser. B (1982) 208-212). DNA encoding the promoter region was derived from the TAKA-amylase genomic clone. The sequence of the promoter and regions upstream to the promoter together with the preregion and the 5'end of the structural gene for the TAKA-amylase is illustrated in Fig. 1.

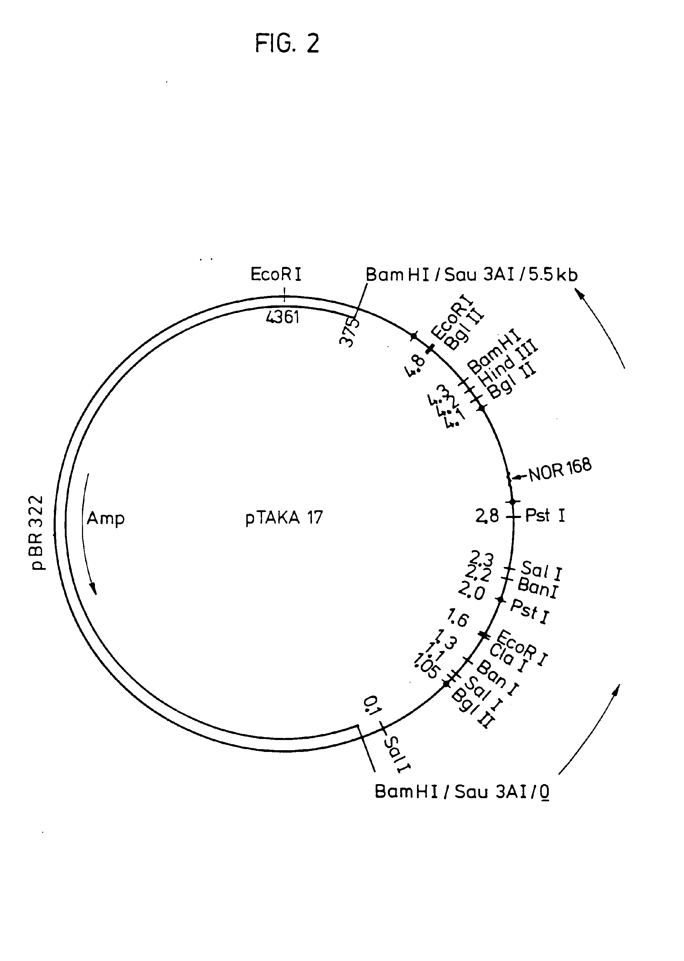

As described in further detail in example 2 a DNA-sequence encoding the TAKA-amylase including the preregion and promoter and upstream activating sequences was derived from a A. oryzae mycelium and inserted in BamHI digested pBR322 to give plasmid pTAKA 17 (see Fig. 2). In pTAKA 17 the A. oryzae derived DNA is shown as a 5.5 kb BamHI/Sau 3AI - BamHI/Sau 3AI fragment, the promoter and upstream activating sequences representing a 2.1 kb fragment starting at position O. The established DNA-sequence of the promoter and upstream activating sequences up to the BglII site is shown in Fig. 1. The promoter ends at nucleotide-1 preceding the Met(1) codon of the TAKA-amylase presequence. The nucleotide sequence encoding the presequence is constituted of 63 nucleotides and the mature TAKA-amylase starts at a position corresponding to nucleotide 64.

From pTAKA 17 the whole promoter sequence including sequences upstream to the promoter or functional parts thereof may be derived by means evident to the person skilled in the art. The promoter sequence may be provided with linkers with the purpose of introducing specific restriction sites facilitating the ligation of the promoter sequence with further DNA, for instance the gene encoding the desired protein product or different preregions (signal peptides).

In the method according to the present invention the sequence from nucleotide -1144 (see Fig. 1) (representing the start of a SalI site) to nucleotide -10 has been used as one example of a well functioning part of the promoter region. In another embodiment of the present invention the nucleotide sequence from nucleotide -1176 to -1 was preceded by the still not sequenced 1.05 kb fragment from pTAKA 17. It is evident for the person skilled in the art that different fragments can be used.

According to one embodiment of the present invention the promoter and upstream activating sequences have the following sequence or a functionally equivalent nucleotide sequence: 
GTCGACGC ATTCCGAATA CGAGGCCTGA TTAATGATTA CATACGCCTC
CGGGTAGTAG ACCGAGCAGC CGAGCCAGTT CAGCGCCTAA AACGCCTTAT
ACAATTAAGC AGTTAAAGAA GTTAGAATCT ACGCTTAAAA AGCTACTTAA
AAATCGATCT CGCAGTCCCG ATTCGCCTAT CAAAACCAGT TTAAATCAAC
TGATTAAAGG TGCCGAACGA GCTATAAATG ATATAACAAT ATTAAAGCAT
 
TAATTAGAGC AATATCAGGC CGCGCACGAA AGGCAACTTA AAAAGCGAAA
GCGCTCTACT AAACAGATTA CTTTTGAAAA AGGCACATCA GTATTTAAAG
CCCGAATCCT TATTAAGCGC CGAAATCAGG CAGATAAAGC CATACAGGCA
GATAGACCTC TACCTATTAA ATCGGCTTCT AGGCGCGCTC CATCTAAATG
TTCTGGCTGT GGTGTACAGG GGCATAAAAT TACGCACTAC CCGAATCGAT
AGAACTACTC ATTTTTATAT AGAAGTCAGA ATTCATAGTG TTTTGATCAT
TTTAAATTTT TATATGGCGG GTGGTGGGCA ACTCGCTTGC GCGGGCAACT
CGCTTACCGA TTACGTTAGG GCTGATATTT ACGTGAAAAT CGTCAAGGGA
TGCAAGACCA AAGTAGTAAA ACCCCGGAAG TCAACAGCAT CCAAGCCCAA
GTCCTTCACG GAGAAACCCC AGCGTCCACA TCACGAGCGA AGGACCACCT
CTAGGCATCG GACGCACCAT CCAATTAGAA GCAGCAAAGC GAAACAGCCC
AAGAAAAAGG TCGGCCCGTC GGCCTTTTCT GCAACGCTGA TCACGGGCAG
CGATCCAACC AACACCCTCC AGAGTGACTA GGGGCGGAAA TTTAAAGGGA
TTAATTTCCA CTCAACCACA AATCACAGTC GTCCCCGGTA TTGTCCTGCA
GAATGCAATT TAAACTCTTC TGCGAATCGC TTGGATTCCC CGCCCCTAGT
CGTAGAGCTT AAAGTATGTC CCTTGTCGAT GCGATGTATC ACAACATATA
AATACTAGCA AGGGATGCCA TGCTTGGAGG ATAGCAACCG ACAACATCAC
ATCAAGCTCT CCCTTCTCTG AACAATAAAC CCCACAG
 representing the sequence from nucleotide -1144 to -10 in Fig. 1.

According to a further embodiment the promoter and upstream activating sequences have the following sequence or a functionally equivalent nucleotide sequence: 
AGATCTGCCC TTATAAATCT CCTAGTCTGA TCGTCGACGC ATTCCGAATA
CGAGGCCTGA TTAATGATTA CATACGCCTC CGGGTAGTAG ACCGAGCAGC
CGAGCCAGTT CAGCGCCTAA AACGCCTTAT ACAATTAAGC AGTTAAAGAA
GTTAGAATCT ACGCTTAAAA AGCTACTTAA AAATCGATCT CGCAGTCCCG
ATTCGCCTAT CAAAACCAGT TTAAATCAAC TGATTAAAGG TGCCGAACGA
GCTATAAATG ATATAACAAT ATTAAAGCAT TAATTAGAGC AATATCAGGC
CGCGCACGAA AGGCAACTTA AAAAGCGAAA GCGCTCTACT AAACAGATTA
CTTTTGAAAA AGGCACATCA GTATTTAAAG CCCGAATCCT TATTAAGCGC
CGAAATCAGG CAGATAAAGC CATACAGGCA GATAGACCTC TACCTATTAA
ATCGGCTTCT AGGCGCGCTC CATCTAAATG TTCTGGCTGT GGTGTACAGG
GGCATAAAAT TACGCACTAC CCGAATCGAT AGAACTACTC ATTTTTATAT
AGAAGTCAGA ATTCATAGTG TTTTGATCAT TTTAAATTTT TATATGGCGG
GTGGTGGGCA ACTCGCTTGC GCGGGCAACT CGCTTACCGA TTACGTTAGG
 
GCTGATATTT ACGTGAAAAT CGTCAAGGGA TGCAAGACCA AAGTAGTAAA
ACCCCGGAAG TCAACAGCAT CCAAGCCCAA GTCCTTCACG GAGAAACCCC
AGCGTCCACA TCACGAGCGA AGGACCACCT CTAGGCATCG GACGCACCAT
CCAATTAGAA GCAGCAAAGC GAAACAGCCC AAGAAAAAGG TCGGCCCGTC
GGCCTTTTCT GCAACGCTGA TCACGGGCAG CGATCCAACC AACACCCTCC
AGAGTGACTA GGGGCGGAAA TTTAAAGGGA TTAATTTCCA CTCAACCACA
AATCACAGTC GTCCCCGGTA TTGTCCTGCA GAATGCAATT TAAACTCTTC
TGCGAATCGC TTGGATTCCC CGCCCCTAGT CGTAGAGCTT AAAGTATGTC
CCTTGTCGAT GCGATGTATC ACAACATATA AATACTAGCA AGGGATGCCA
TGCTTGGAGG ATAGCAACCG ACAACATCAC ATCAAGCTCT CCCTTCTCTG
AACAATAAAC CCCACAGAAG GCATTT
 representing the sequence from nucleotide -1176 to -1 in Fig. 1.

According to a further aspect of the present invention the latter sequence may be preceded by the 1.05 kb unsequenced upstream region from pTAKA 17 (position 0 to 1.05 in Fig. 2).

The terminators and polyadenylation sequences may be derived from the same sources as the promoters. Enhancer sequences may also be inserted into the construction.

The expressed product may be accumulated within the cells requiring disruption of the cells to isolate the product. To avoid this further process step and also to minimize the amount of possible degradation of the expressed product within the cells it is preferred that the product is secreted from the cells. For this purpose the gene for the desired product is provided with a preregion ensuring effective direction of the expressed product into the secretory pathway of the cell. This preregion which might be a naturally occuring signal or leader peptide or functional parts thereof or a synthetic sequence providing secretion is generally cleaved from the desired product during secretion leaving the mature product ready for isolation from the culture broth.

The preregion may be derived from genes for secreted proteins from any source of organism.

According to the present invention the preregion may be derived from a glucoamylase or an amylase gene from an Aspergillus species, an amylase gene from a Bacillus species, a lipase or proteinase gene from Rhizomucor miehei, the gene for the α-factor from S. cerevisiae or the calf prochymosin gene. More preferably the preregion is is derived from the gene for A. oryzae TAKA amylase, A. niger neutral α-amylase, A. niger acid-stable α-amylase, B. licheniformis α-amylase, the maltogenic amylase from Bacillus NCIB 11837, B. stearothermophilus α-amylase or B. licheniformis subtilisin. An effective signal sequence is the A. oryzae TAKA-amylase signal, the Rhizomucor miehei aspartic proteinase signal and the Rhizomucor miehei lipase signal.

The TAKA-amylase signal has the following sequence

ATGATGGTCGCGTGGTGGTCTCTATTTCTGTACGGCCTTCAGGTCGCGGCACCT
MetMetValAlaTrpTrpSerLeuPheLeuTyrGlyLeuGlnValAlaAlaPro

GCTTTGGCT
AlaLeuAla

The gene for the desired product functionally linked to promoter and terminator sequences may be incorporated in a vector containing the selection marker or may be placed on a separate vector or plasmid capable of being integrated into the genome of the host strain. As used herein the expression "vector system" includes a single vector or plasmid or two or more vectors or plasmids which together contain the total DNA-information to be integrated into the host genome. Vectors or plasmids may be linear or closed circular molecules. According to a preferred embodiment of the present invention A. oryzae is cotransformed with two vectors, one including the selection marker and the other comprising the remaining foreign DNA to be introduced in the host strain, including promoter, the gene for the desired product and transcription terminator and polyadenylation sequences.

Normally the A. oryzae transformants are stable and can be cultured in the absence of a selection pressure. If the transformants turn out to be unstable the selection marker may be used for selection during cultivation. The transformed cells are then cultured under a selection pressure corresponding to the marker in question.

The present invention provides for a method for production of high yields of many different polypeptide or protein products in A. oryzae. A. oryzae has for years been used in commercial scale for the production of for instance the TAKA-amylase enzyme and proteolytic enzymes and accordingly fermentation technology for this microorganism is well developed and the microorganism is approved for use in the food industry. The present invention offers the possibility of using A. oryzae in the industrial production of high amounts of in principle any polypeptide or protein product. Examples of such products are chymosin or prochymosin and other rennets, proteases, amyloglucosidases, acid stable amylases from Aspergillus, fungal lipases or prokaryotic lipases, and thermostable bacterial and fungal amylases.

The present invention is illustrated by means of the production of prochymosin, Rhizomucor miehei aspartic proteinase, TAKA-amylase and a lipase from Rhizomucor miehei.

The genes for these enzymes were obtained from cDNA libraries or genomic libraries as described in further detail in the following.

### EXAMPLES

Plasmids used as starting materials in the following examples are as follows:
- p285:: (ATCC No. 20681)
- pCAMG91:: Boel et al. EMBO Journal 3 (1984), 1581-1585.
- pIC19R:: Marsh et al. Gene 32 (1984), 481-485
- pSal43:: Berse et al. Gene 25 (1983), 109-117 John & Peberdy, Enzyme Microb. Technol.
6 (1984), 386-389.
- p3SR2:: J.M. Kelly and M.J. Hynes, EMBO Journal 4 (1985), 475-479.
- pBR322:: Bolivar F. et al., Gene 2 (1977), 95-113.
- pBR327:: Covarrubias L. et al., Gene 13 (1981), 25-35.
- pUC9, pUC13, and pUC19:: Vieira et al., Gene 19 (1982), 259-268 and Messing, Meth. in Enzymology 101 (1983), 20-27.

The strains used are as follows:
- A. niger:: ATCC 1015, ATCC 10582
- A. oryzae:: ATCC 20423, IFO 4177, ATCC 1011, ATCC 9576, ATCC 14488-11491, ATCC 11601 and ATCC 12892.
- E . coli :: MC1000 (Casabadan, M.J. and Cohen, S.N., J.Mol.Biol. 138, 179-207) (NCIB 11956)
- Rhizomucor miehei:: CBS 370.65

### Example 1

### Preparation of plasmid 285'proC containing the prochymosin gene

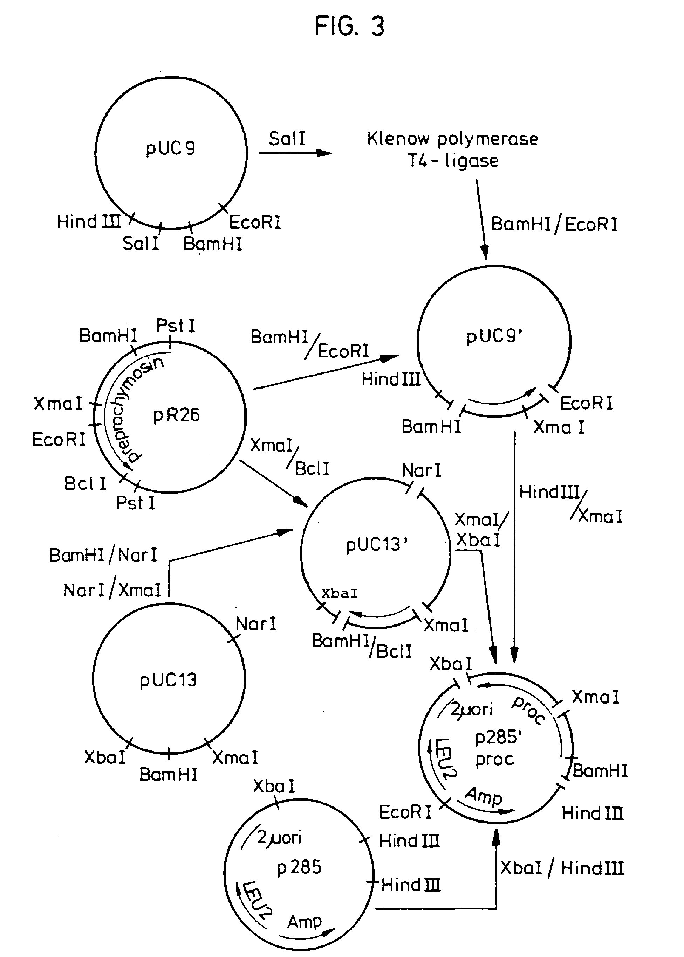

The preprochymosin gene was isolated from a calf stomach cDNA library and inserted into the FstI site of pBR322 by G-C tailing (Chirgwin et al., Biochemistry 18 (1979), 5294 and Truelsen et al., Nucleic Acids Res. 6 (1979), 3061) to obtain pR26. pUC9 was cut with SalI and filled cut with Klenow polymerase and ligated with T4 ligase. The resulting plasmid was cut with BamHI-EcoRI and the 2.7 kb large fragment was ligated with a 0.47 kb BamHI-EcoRI fragment from pR26 containing the N-terminal end of the prochymosin gene to create pUC9'. pUC9' contains a HindIII site N-terminally of the prochymosin gene. pUC13 was cut with BamHI-NarI and NarI-XmaI and the large respective small fragments were ligated with a 0.64 kb XmaI-BclI fragment of pR26 containing the C-terminal end of the prochymosin gene to obtain plasmid pUC13'. pUC13' contains an XbaI-site C-terminally of the prochymosin gene. A 0.65 kb XmaI-XbaI fragment of pUC13' was ligated with a 0.46 kb HindIII-XmaI fragment of pUC9' and a 11 kb XbaI-HindIII fragment of p285 to create plasmid p285' proC containing the prochymosin gene as illustrated in fig. 3.

### Example 2

### Cloning of the A. oryzae TAKA-amylase A gene

### Isolation of a partial cDNA clone

From A. oryzae Hw 325 grown on potato starch, mRNA was prepared according to Kaplan et al., Biochem. J. 183, (1979) 181 - 84. A partial cDNA clone containing 1050 bp of the TAKA-amylase gene was obtained by specific priming of mRNA with a 14-mer oligonucleotide mixture: 
5'GG^ATT^ATC^ATG^ATT 3'(NOR-168),
    G   G   G   G
 complementary to the coding sequence for amino acids 295 - 299 in TAKA-amylase (Toda et al., Proc. Japan Acad. 58, Ser. B, (1982) 208 - 12). Cloning procedure was according to Gubler & Hoffmann, Gene 25, (1983) 263 - 69. Sequencing at the ends and in the middle of the cDNA clone demonstrated presence of sequences corresponding to the amino acid sequence of TAKA-amylase.

### Isolation of genomic clones

Mycelium from A. oryzae Hw 325 was harvested and processed for preparation of DNA according to the method used for A. niger described by Boel et al. supra. Restriction fragments of 3 - 10 kb, generated by partial digestion with Sau3A, were ligated with BamHI digested, dephosphorylated pBR322 (New England Biolabs). 50,000 recombinants were screened with oligonucleotide probe NOR-168 (see above), and 7 were found to contain DNA coding for TAKA-amylase. One clone was chosen for further use of the promoter region, having 2.1 kb upstream of the mRNA-start. Restriction map for plasmid pTAKA 17 is outlined in Fig. 2. pTAKA was deposited with Deutsche Sammlung von Mikroorganismen (DSM), Griesebachstrasse 8, D-3400, Gottingen, on February 23, 1987 and accorded the reference number DSM 4012 DSM being an international depository authorized under the Budapest Treaty of 1977, affords permanence of the deposit and accessibility thereto by the public in accordance with rules 9 and 11, respectively, of the above treaty.

### Example 3

### Construction of a Rhizomucor miehei cDNA library

The phycomycete fungus Rhizomucor miehei (for a morphological and taxor.omical description of this species see: Schipper, M.A.A. On the genera Rhizomucor and Parasitella. In: Studies in mycology, Institute of the Royal Netherlands Academy of Sciences and Letters. No. 17 (1978), 53-71) secretes an acid proteinase (Rhizomucor miehei proteinase, in the following abreviated to RMP) which is widely used for clotting of milk in cheese production. In order to obtain cDNA recombinant clones of this protein in E. coli, total RNA was extracted from homogenized R. miehei mycelium as described by Boel et al. (EMBO J., 3: 1097-1102, 1984) and Chirgwin et al., (Biochemistry (Wash.), 18, 5294-5299, 1979). Poly(A)-containing RNA was obtained by two cycles of affinity chromatography on oligo(dT)-cellulose as described by Aviv and Leder (PNAS, USA, 69, 1408-1412, 1972). Oligo(dT) primed complementary DNA was synthesized and made doublestranded according to Gubler and Hoffman (Gene, 25, 263-269, 1983). Doublestranded cDNA was tailed with dCTP and terminal deoxynucleotidyl transferase as described by Roychoudhury et al. (Nucleic Acids Res, 3, 101-106, 1976). The plasmid pBR327 was linearized with PstI and tailed with dGTP. The oligo(dC) tailed dscDKA was annealed to this oligo(dG) tailed vector as described by Peacock et al. (Biochim.Biophys.Acta, 655, 243-250, 1981) and used to transform a hsdR⁻, M⁺ derivative of E. coli MC1000 (Casadaban and Cohen, J. Mol.Biol., 138, 179-207, 1980) to generate recombinant clones.

### Identification of RMP specific cDNA recombinants

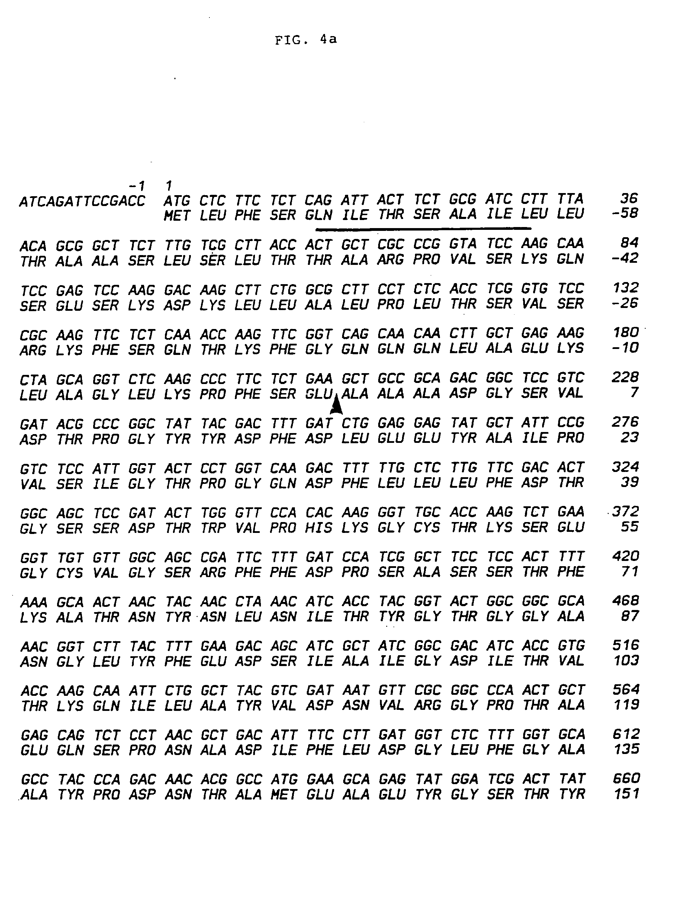

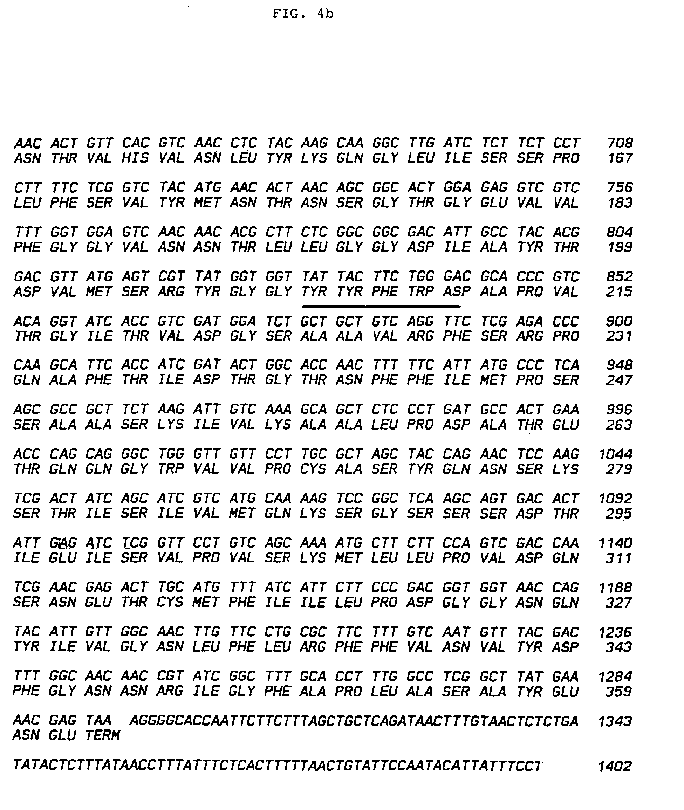

A mixture of 16 heptadecamer oligodeoxyribonucleotides 
    A     A  A  A
d(GC TCCCA AA TA TA),
    G     G  G  G
 one of which is complementary to RMP mRNA in the region coding for Tyr-Tyr-Phe-Trp-Asp-Ala (Bech and Foltmann, Neth-milk Dairy J. 35: 275-280, 1981) was synthesized on an Applied Biosystems, Inc. DNA synthesizer and purified by polyacrylamide gel electrophoresis. Approximately 10,000 E. coli recombinants from the Rhizomucor miehei cDNA library were transferred to Whatman 540 paper filters. The colonies were lysed and immobilized as described by Gergen et al. (Nucleic Acids Res. 7, 2115-2135, 1979). The filters were hybridized with the ³²P-labelled RMP-specific heptadecamer-mixture as described by Boel et al. (EMBO J. 3, 1097-1102, 1984). Hybridization and washing of the filters were done at 40°C, followed by autoradiography for 24 hours with an intensifier screen. Miniprep plasmid DNA was isolated from a hybridizing colony, pRMP1016, by standard procedures (Birnboim and Doly, Nucleic Acids Res., 7, 1513-1523, 1979), and the DNA sequence of the cDNA insert was established by the procedure of Maxam and Gilbert (Methods Enzymol. 65, 499-560, 1980). pRMP1016 was shown to contain part of the 5' untranslated end of the mRNA and then extending through regions encoding a 69 amino acid-long preproregion and 300 amino acids into the mature part of the RMP protein. Since pRMP1016 did not contain any insert corresponding to the complete 3' end of the RMP mRNA, the cDNA library was rescreened with a ³²P nick-translated 3' specific restriction fragment from clone pRMP1016, whereby clone pRMP2931 was isolated. This clone contains part of the 3' untranslated region and an open reading frame with the 270 triplets encoding the carboxyterminal part of the RMP protein. pRMP1016 and pRMP2931, therefore, have extensive overlap, and the combined sequence of the two clones gives the sequence of the R. miehei preproRMP cDNA. A total of 1416 nucleotides was sequenced between the G:C tails resulting from the cDNA cloning procedure. The established DNA sequence is shown in Fig. 4a and b together with the deduced amino acid sequence of a precursor to RMP. In Fig. 4a and 4b the horizontal line indicates the position of a synthetic oligo mixture used for cDNA library screening. An arrow shows the position where processing occurs in maturation of native RMP. Nucleotides are numbered from the first base in the initiating Met-codon and amino acids are numbered from the first residue in the mature RMP. From this cDNA sequence it can be concluded that RMP is synthesized as a 430 amino acids long precursor with a propeptid of 69 amino acids. A putative signal peptidase processing site (von Heijne, Eur.J.Biochem. 133, 17-21, 1983) in this precursor could be between Ala(-48) and Arg(-47), and the mature RMP will be generated by autoproteolytic cleavage between Glu-1 and Ala(+1). The cDNA deduced amino acid sequence of RMP is in good agreement with the previously published partial amino acid sequence (Bech and Foltmann, Neth-Milk Dairy J. 35: 275-280, 1981).

To facilitate further construction work with the RMP cDNA's, a HindIII linker was inserted at a BanI site just 3' to the TAA-termination codon identified in clone pRMP2931 as follows: 25µg pRMP2931 was digested with PstI to obtain the RMPcDNA insert. This insert was purified by 1% agarose gel electrophoresis, electroeluted from the gel, purified by phenol and cloroform extractions and precipitated with NaCl and ethanol. This fragment that encodes the 3' half of the RMP, was digested with BanI and the BanI cohesive restriction site ends were filled in with a mixture of the four dNTP's and the Klenow fragment of E. coli DNA polymerase. To these filled-in ends were added HindIII linkers in a T4-DNA ligase reaction. The ligation reaction mixture was extracted with phenol and chloroform and the DNA was precipitated with 4M NH₄⁺ acetate/ethanol. The purified DNA was digested with an excess of HindIII enzyme, and a 380 bp fragment was purified on a 6% polyacrylamide gel. This fragment that contains the 3' end of the RMP open reading frame plus the TAA termination codon was ligated to a HindIII digested and alkaline phosphatase treated pIC19R. The ligation mixture was used to transform competent E. coli cells, and transformants were selected on ampicillin containing agar plates. Plasmid DNA was purified from transformants and correct recombinants were identified by restriction endonuclease digestions and agarose gel electrophoresis. From one such correct recombinant, pRMP3', a 210 bp BglII/HindIII fragment was isolated by 6% polyacrylamide gel electrophoresis. This fragment contains the 3'end of RMP cDNA from the BglII site at amino acids 297-299 and extending through the TAA-termination codon to the inserted HindIII linker.

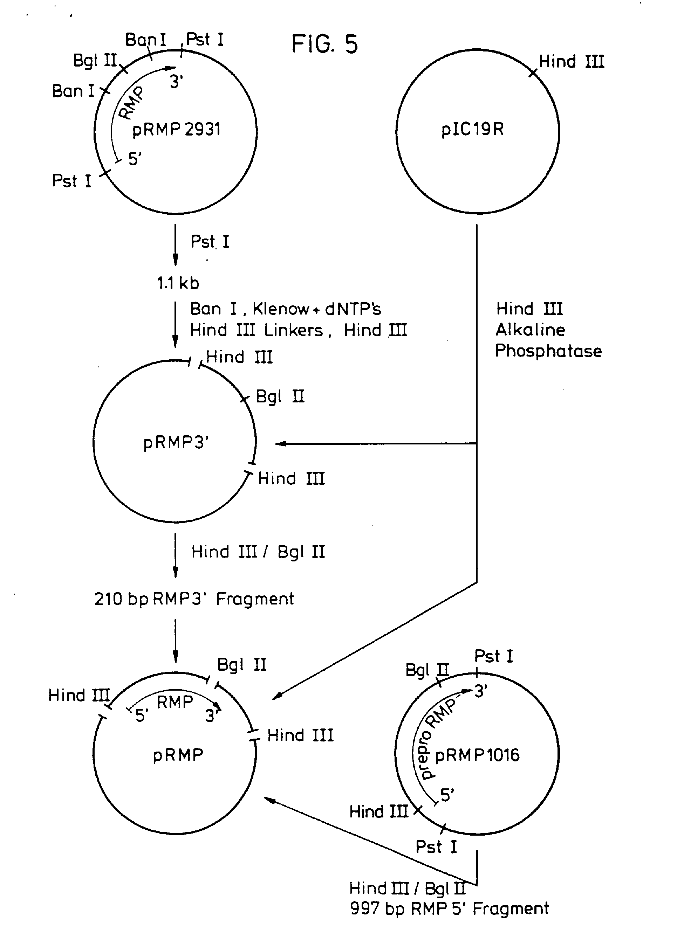

The 5' part of the RMP cDNA was isolated from pRMP 1016 as a 997 bp HindIII/BglII fragment by 1% agarose gel electrophoresis. The HindIII site is located in the RMP-DNA at a position corresponding to the residues -36, -35 in the prosegment. This 997 bp 5' fragment was ligated to the 210 bp 3' fragment in a HindIII digested and phosphatase treated pIC19R. With this ligation mixture, recombinants were obtained from E. coli and a correct plasmid, pRMP, with the 5' part of RMP joined to the 3' part was identified by restriction enzyme analysis. The construction of pRMP is illustrated in Fig. 5. pRMP does not encode the RMP preregion and the 5' half of the prosegment.

### Example 4

### Construction of an Aspergillus expression vector designed to obtain secretion of active RMP

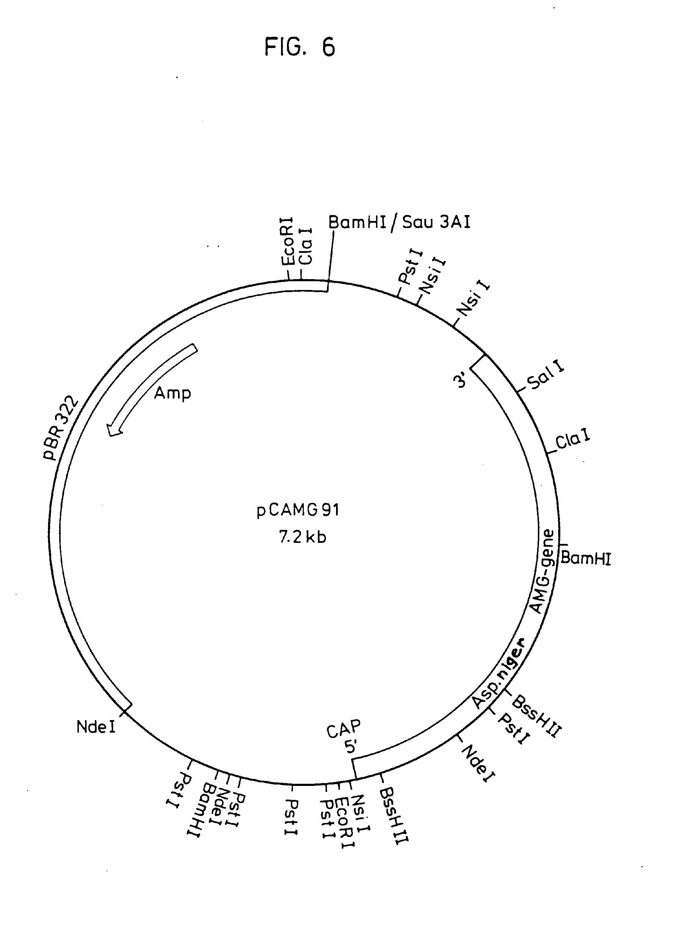

In this example a plasmid was constructed designed to express RMP under control of the glucoamylase promoter, signal and terminator sequences. The glucoamylase promoter and terminator sequences were derived from the glucoamylase genomic gene cloned in vector pCAMG91. The construction of pCAMG91 is described by Boel et al. (EMBO Journal 3 (1984), 1581-1585) and an endonuclease restriction map of plasmid pCAMG91 is shown in Fig. 6.

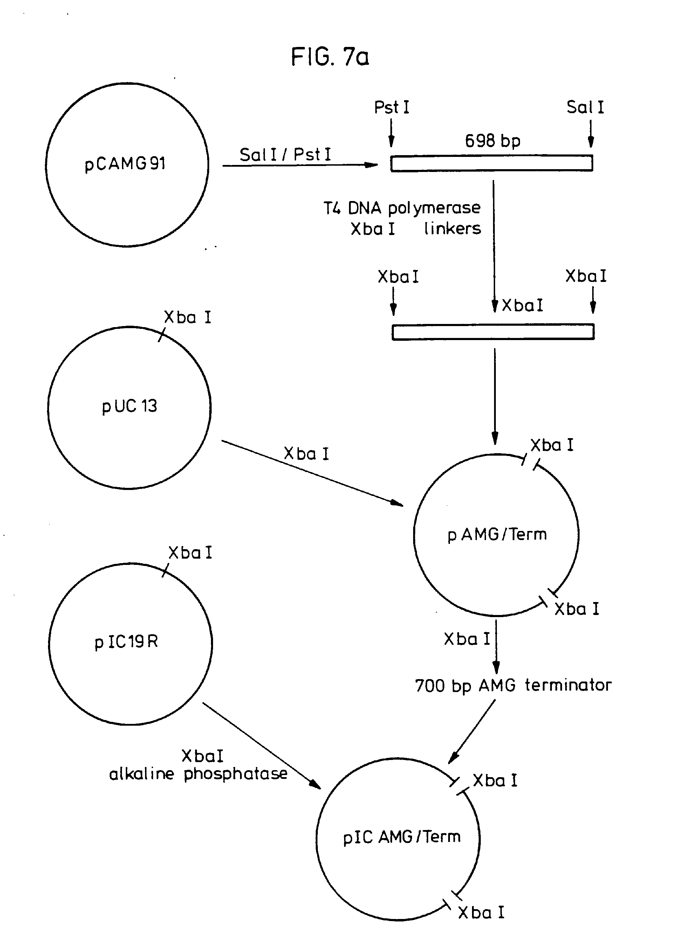

pCAMG91 was digested with SalI and PstI restriction endonucleases. From such a digest a 698 bp fragment was isolated on an agarose gel. This SalI-PstI fragment contains the region encoding the 140 bp 3' untranslated part of the glucoamylase mRNA plus 540 bp 3' to the poly(A)-addition site. This 3'fragment was treated with T4-DNA polymerase to "blunt end" the restriction sites before the addition of XbaI linkers and digestion with XbaI restriction enzyme. This 3' end of the glucoamylase gene was ligated to pUCl3 linearized with XbaI to create plasmid pAMG/Term containing the glucoamylase gene poly(A) addition region. The construction of pAMG/Term is illustrated in Fig. 7a.

The 3'end of the A. niger glucoamylase gene was obtained as a 700 bp XbaI fragment from pAMG/Term. This terminator fragment was ligated to XbaI digested and phosphatase treated pIC19R. With this ligation mixture recombinants were obtained from E. coli and a correct plasmid, pICAMG/Term, with the 5'end of the teminator fragment facing the HindIII site of the multiple cloning site of the pICl9R vector was identified by restriction enzyme analysis. The construction of pICAMG/Term is illustrated in Fig. 7a. From pICAMG/Term the glucoamylase terminator (AMG terminator) region was isolated as a 750 bp HindIII/ClaI restriction fragment by 1% agarose gel electrophoresis. From pCAMG91 the glucoamylase promoter (AMG promoter) was isolated together with regions encoding the glucoamylase signal peptide, hexapeptide-prosegment and the pBR322 ampicillin resistence gene (Amp) as a 3.5 kb ClaI/BssHII fragment by 1% agarose gel electrophoresis. A synthetic BssHII/HindIII linker was prepared from two synthetic 31'mer oligonucleotides synthesized on an Applied Biosystems Inc. DNA-synthesizer. The synthetic linker has the following structure:

R V S K Q S E S K D
CGCGTAAGTAAGCAGAGCGAGAGCAAGGATA
ATTCATTCGTCTCGCTCTCGTTCCTATTCGA

This linker was used in a ligation reaction with the 3.5 kb glucoamylase promoter containing fragment and the 750 bp glucoamylase terminator containing fragment. The ligation mixture was used to transform E. coli and a correct recombinant, p673, was identified by restriction endonuclease digestion. The isolated p673 is a HindIII cloning vector, into which an appropriate HindIII cDNA fragment can be inserted between the glucoamylase hexapeptide prosegment and the glucoamylase transcription terminator region. The inserted cDNA will be under transcriptional control by the glucoamylase promoter, and secretion of the translated fusion product will be directed by the glucoamylase signal peptide plus the glucoamylase hexapeptide prosegment. p673 was digested with HindIII, treated with alkaline phosphatase and ligated with a 1.2 kb HindIII fragment purified from a digest of pRMP.

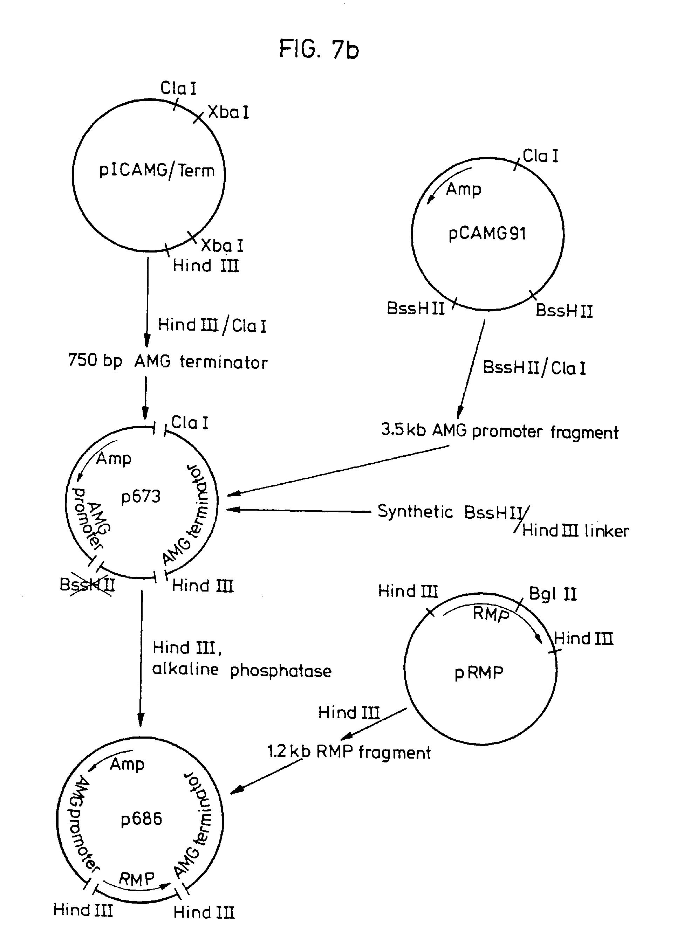

The ligation mixture was used to transform E. coli and a recombinant, p686, with the RMP cDNA inserted in the correct orientation to achieve RMP expression was isolated and characterized by restriction endonuclease digestions. p686 encodes a RMP precursor with the following structure: glucoamylase signal peptide, glucoamylase hexapropeptide, amino acids - 45 to -1 of the propeptide from RMP, 361 amino acids of mature RMP. The construction of p686 is illustrated in Fig. 7b.

### Example 5

In a preferable embodiment of the present invention the open reading frame of preproRMP should be inserted in an expression plasmid under control of the promoter from the glucoamylase gene from A. niger or the TAKA-amylase gene from A. oryzae. To do this, a BamHI restriction endonuclease site was inserted just 5' to the initiating methionine codon of the signal peptide of preproRMP by the following steps. pRMP1016 was digested with DdeI which cuts in the cDNA at a position corresponding to amino acid residues Ser(- 66) and Gln(-65), and with HindIII which cuts in the cDNA at a position corresponding to amino acid residues Lys(-36) and Leu(-35). The resulting 89 bp DdeI/HindIII fragment was purified on a 8% polyacrylamide gel, electroeluted and ethanolprecipitated after phenol and CHCl₃ extractions. A synthetic DNA fragment with the following sequence was synthesized as two oligonucleotides on an Applied Biosystems Inc. DNA synthesizer:

          M   L   F   S
GATCCACCATGCTGTTCTC    oligo 697/698
    GTGGTACGACAAGGAAGT

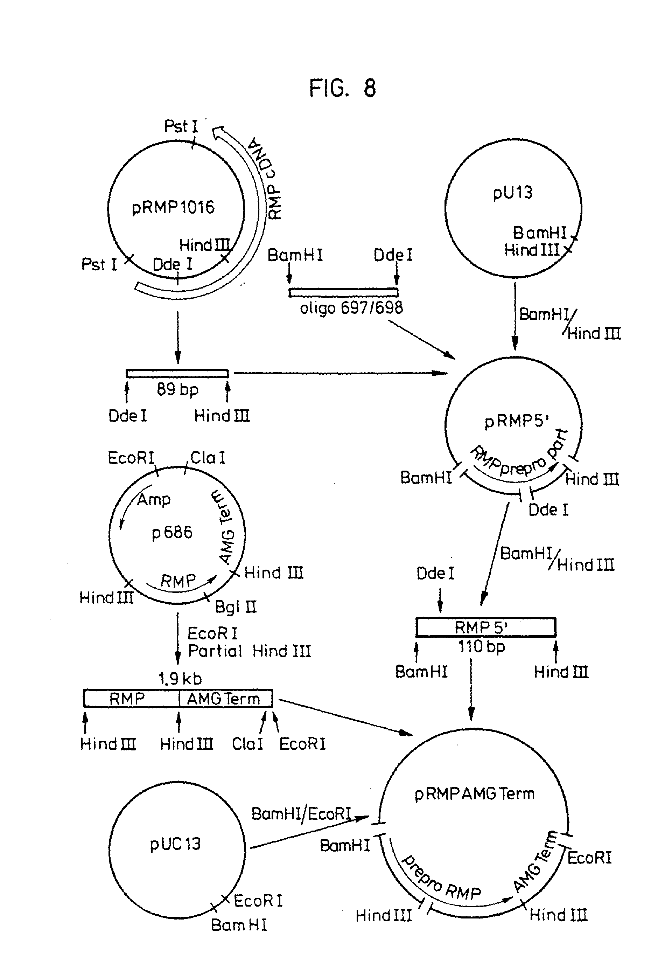

This fragment has a BamHI cohesive end 5' to the initiating Met-codon and a DdeI cohesive end in the 3' end. The two oligonucleotides were kinased with ATP and T4 polynucleotide kinase, annealed to each other and then ligated to the 89 bp DdeI/HindIII RMP fragment purified from pRMP1016 in a BamHI/HindIII digested pUCl3 vector. The ligation mixture was used to transform E. coli cells, and correct recombinants were identified by restriction enzyme digestions on miniprep purified plasmids. Correct recombinant plasmids were sequenced to verify the sequence of the oligonucleotides used. One such correct plasmid pRMP5' was digested with BamHI and HindIII, and a 110 bp BamHI/HindIII fragment with the initiating Met codon, RMP signal peptide and part of the RMP prosegment was purified by 10% polyacrylamide gel electrophoresis. The fragment was electroeluted, phenol and CHCl₃ extracted and ethanol precipitated. The rest of the RMP open reading frame and the AMG terminator sequences were obtained from plasmid p686 after digestion with EcoRI and partial HindIII digestion. Hereby a 1.9 kb fragment was released and this fragment was purified by agarose gel electrophoresis, electroelution, phenol and CHCl₃ extraction before ethanol precipitation. This 1.9 kb fragment was ligated to the 110 bp BamHI/HindIII fragment from pRMP5' in a pUCl3 vector that had been digested with BamHI and EcoRI. The ligation mixture was used to transform E. coli cells and correct recombinants were identified by restriction enzyme digestions on miniprep purified plasmids. One such correct recombinant was pRMPAMGTerm. The construction of pRMPAMGTerm is illustrated in Fig. 8.

### Example 6

### Construction of an Aspergillus expression vector designed to obtain secretion of active RMP in A. oryzae by means of the Aspergillus niger glucoamylase promoter

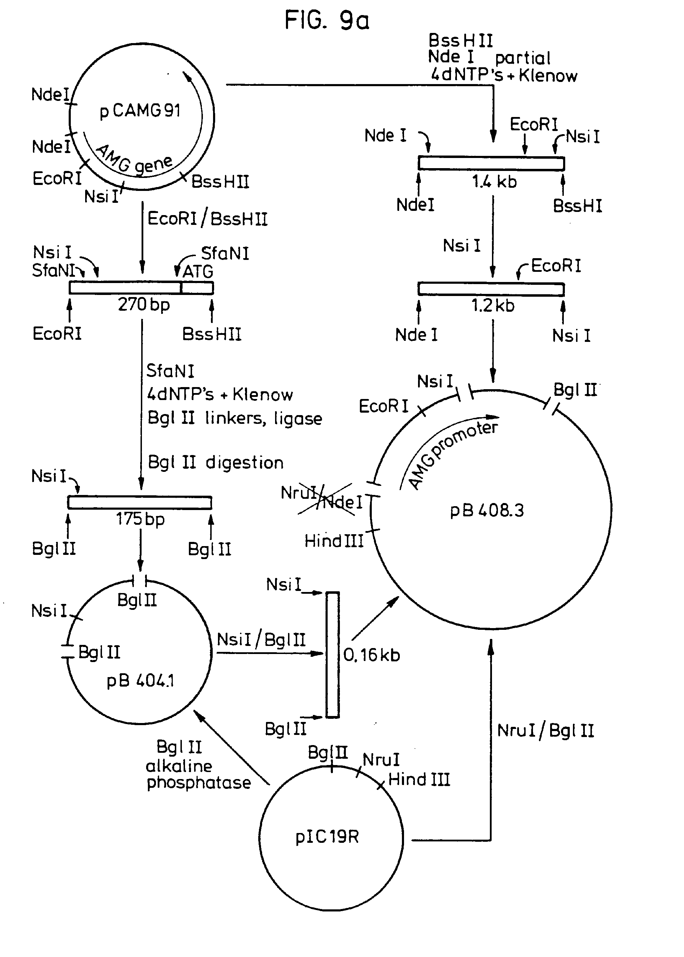

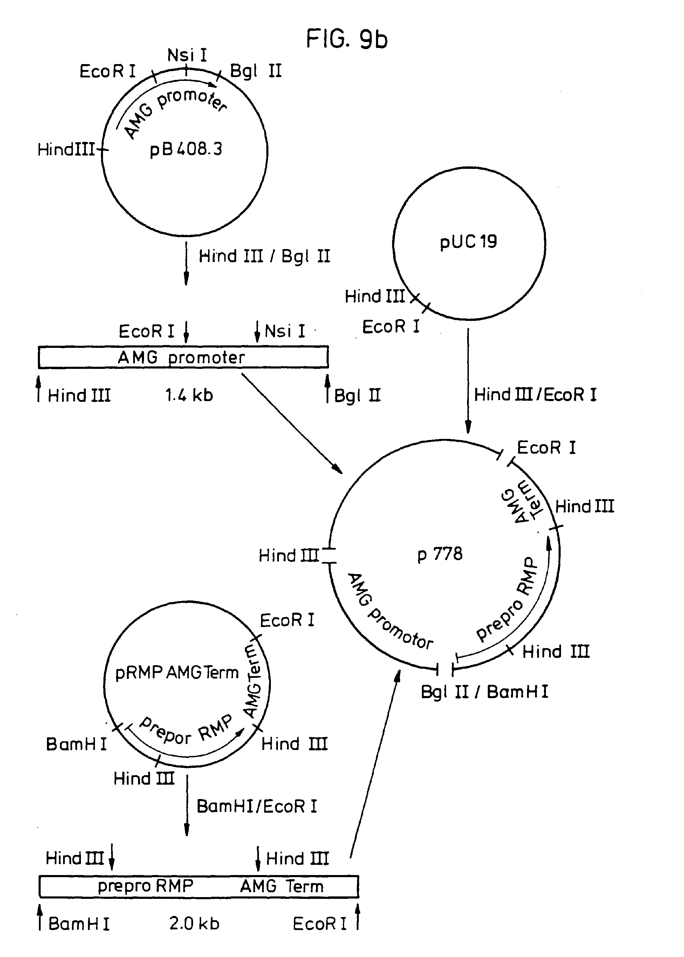

The glucoamylase promoter was isolated as follows. 25 µg of pCAMG91 was digested with EcoRI and BssHII restriction endonucleases. After this double digestion a 270 bp DNA fragment could be isolated by agarose gel electrophoresis. This fragment covers part of the promoter region, the 5' untranslated region and the signal peptide of the glucoamylase gene (AMG gene). After electroelution of the DNA from the agarose gel, the fragment was purified by phenol and CHCl₃ extractions before ethanol precipitation. The 270 bp long fragment was then digested with SfaNI. This enzyme has a cleavage site just 5' to the initiating ATG methionine codon of the glucoamylase gene. After complete digestion, the DNA was treated with the large fragment (Klenow) of DNA polymerase I and all four dNTP's to generate blunt ends on the DNA. To this DNA was added BglII linkers with DNA ligase, and the DNA was digested with an excess of BglII restriction enzyme. After separation of the DNA fragments on a 10% polyacrylamide gel, a 175 bp BglII fragment could be isolated by electroelution. This fragment has a BglII linker inserted in a position corresponding to the SfaNI restriction site just 5' to the initiating methionine codon. This piece of DNA was ligated to a BglII digested alkaline phosphatase treated pIC19R vector, and the ligation mixture was used to transform E. coli cells. Among the resulting transformants correct plasmids were identified by restriction enzyme digestions on miniprep plasmids. One such correct plasmid pB404.1 was digested with NsiI and BglII to liberate a 0.16 kb fragment which contained the 5' untranslated region of the glucoamylase gene together with approximately 100 bp of the 3' part of the promoter region. This fragment was purified by polyacrylamid gel electrophoresis, electroeluted, phenol and CHCl₃ extracted and ethanol precipitated. To join this fragment to the remaining part of the glucoamylase promoter region from pCAMG91, the following steps were carried out. 25 µg pCAMG91 was digested with BssHII, and then further partially digested with NdeI. After filling in the fragment ends with all four dNTP's and the Klenow fragment of DNA polymerase, a 1.4 kb DNA fragment was isolated on a 1% agarose gel. This fragment contained all of the promoter region together with the 5' untranslated region and the signal peptide encoding region. The fragment was electroeluted, phenol and CHCl₃ extracted and ethanol precipitated to concentrate the DNA. After digestion with NsiI, the DNA was run on a 1% agarose gel, and a 1.2 kb NdeI - NsiI fragment was isolated by electroelution. This DNA had been given a blunt end at the NdeI site in a previous reaction and it was now ligated to the 0.16 kb NsiI - BglII fragment from pB401.1 in a NruI - BglII digested pIC19R vector. The ligation mixture was used to transform E. coli cells, and among the resulting transforments correct recombinants were identified by restriction enzyme digestions of miniprep plasmids. One such correct recombinant, pB408.3 was digested with HindIII and BglII, and the glucoamylase (AMG) promoter was isolated as a 1.4 kb fragment on a 1% agarose gel. The fragment was electroeluted, phenol and CHCl₃ extracted and ethanol precipitated. This glucoamylase promoter fragment was then ligated to a 2.0 BamHI-EcoRI fragment from pRMPAMGTerm (see example 5) in a HindIII-EcoRI digested pUC19 vector. The ligation mixture was used to transform E. coli cells, and among the resulting transformants correct recombinants were identified by restriction enzyme digestions of miniprep plasmids. One such correct recombinant p778 was grown in large scale for isolation of recombinant plasmid and the plasmid preparation was purified by CsCl/Ethidium bromide centrifugation. This plasmid directs the synthesis of RMP under control of the glucoamylase promoter and terminator sequences. The construction of p408.3 is illustrated in Fig. 9a and the construction of p778 is illustrated in Fig. 9b.

### Example 7

### Construction of an Aspergillus expression vector designed to obtain secretion of active RMP by means of the Aspergillus oryzae TAKA-amylase promoter

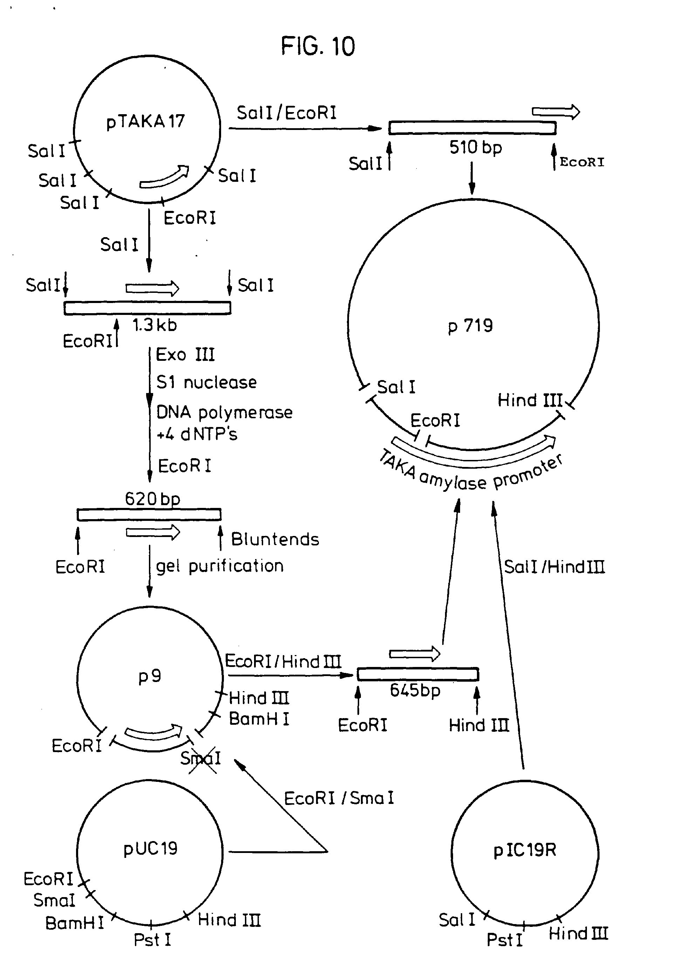

50 µg of plasmid pTAKA17 (see example 2) which contains the Aspergillus oryzae TAKA-amylase genomic gene was digested with SalI. This enzyme has a restriction site in the genomic DNA at a position corresponding to amino acid residue 26 of the mature TAKA-amylase. Another SalI restriction site is located app. 1300 nucleotides upstream to this position that is in the 5'end of the upstream promoter region. After SalI digestion this 1300 bp promoter containing fragment was purified by agarose gel electrophoresis and the DNA was purified by phenol and CHCl₃ extractions and ethanol precipitated. The DNA was then dissolved in exonuclease III buffer and digested with exonuclease III according to Henikoff, S. (Gene, 28: 351-359, 1984). The reaction was stopped to obtain approximately 130 bp deletions in each end of the DNA. The deletion of app. 130 bp from the SalI site of the coding region of the TAKA-amylase gene in this way gives the opportunity to introduce multiple cloning site linkers just upstream of the initiating methionine codon. The exonuclease III treated DNA was digested with Sl nuclease according to Henikoff, S. (Gene, 28: 351-359, 1984) and precipitated with ethanol after phenol and CHCl₃ extractions. Repair of the Sl nuclease treated DNA to obtain ligatable blunt ends were done with all four dNTP's and the Klenow fragment of DNA polymerase I according to Henikoff, S., (Gene, 28: 351-359, 1984). The DNA was digested with EcoRI which cuts once in the 1300 bp SalI fragment to generate two groups of fragments. One group was about 380 bp long and represented upstream regions while the other group was about 620 bp long and contained the promoter region. These groups of EcoRI digestion products were separated on an agarose gel, and the app. 620 bp long DNA fragments were electroeluted and ligated to an EcoRI/SmaI digested pUC19 vector. The ligation mixture was used to transform competent E. coli cells, and miniprep plasmid DNA was isolated from the recombinants. These deletion mutants were characterized by restriction enzyme digestions to identify plasmids with deletion end points just 5' to the initiating methionine codon. A few candidates with the desired characteristics were sequenced and a mutant (p9) that had 9 bp deleted 5' to the A in the ATG-methionine codon was chosen for further constructions. p9 was digested with EcoRI and HindIII, and a 645 bp TAKA-amylase promoter containing fragment was isolated by agarose gel electrophoresis, phenol and CHCl₃ extracted and precipitated with ethanol. pTAKA17 was digested with SalI and EcoRI and a 510 bp fragment containing the TAKA-amylase-promoter upstream regions was isolated by agarose gel electrophoresis, phenol and CHCl₃ extracted and precipitated with ethanol. These two promoter regions were ligated to each other and to a pICl9R vector, that had been digested with SalI and HindIII. The ligation mixture was used to transform E. coli cells, and correct recombinants were identified by restriction enzyme digestion of plasmids extracted as minipreps. In one such recombinant p719 the TAKA-amylase promoter region from Aspergillus oryzae is found as a 1.1 kb portable fragment that can be excised by a number of various restriction enzyme digests. The construction of p719 is illustrated in Fig. 10.

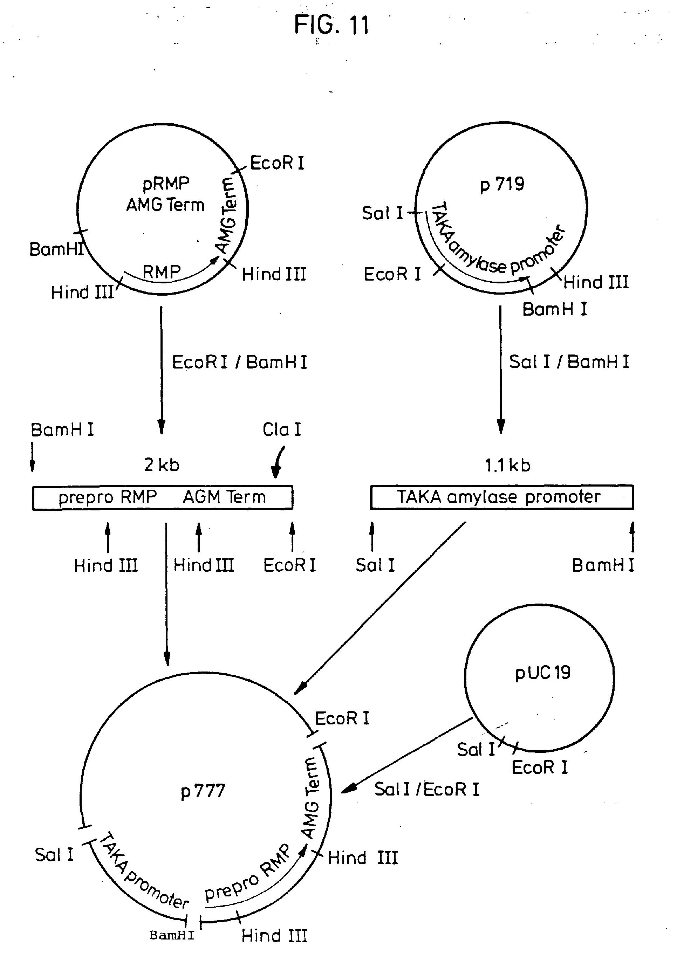

From pRMPAMGTerm the preproRMP open reading frame and glucoamylase terminator region (AMGTerm) was isolated as a 2 kb fragment after digestion with BamHI and EcoRI. This fragment was purified by agarose gel electrophoresis, phenol and CHCl₃ extractions and then concentrated by ethanol precipitation. The promoter from the TAKA-amylase from A. oryzae was now isolated as a 1.1 kb fragment obtained after digestion of p719 with SalI and BamHI. This fragment was purified by agarose gel electrophoresis, phenol and CHCl₃ extractions and then ethanol precipitated. The 1.1 kb promoter fragment was ligated to the 2 kb BamHI/EcoRI fragment from pRMPAMGTerm in a pUCl9 vector, that had been digested with Sail and EcoRI. The ligation mixture was used to transform E. coli cells and among the resulting transformants correct recombinants were identified with restriction enzyme digestion of miniprep plasmids. One such correct recombinant p777 was grown in large scale for the isolation of recombinant plasmid, and the plasmid preparation was purified by CsCl/Ethidium bromide centrifugation. The construction of p777 is illustrated in Fig. 11.

### Example 8

### Construction of an Aspergillus expression vector designed to obtain secretion of the Rhizomucor miehei lipase under control of the Aspergillus oryzae TAKA-amylase promoter

### Construction and identification of a lipase cDNA clone in E. coli

In order to obtain information which allows the construction of a specific oligonucleotide probe, a partial sequence determination was carried out on the purified Rhizomucor miehei lipase (Moskowitz, G.J. et al., J.Agric. Food Chem., 25 (1977), 1146-1150). In the following text the abbreviation RML is used for the Rhizomucor miehei lipase.The supernatant from a culture broth of Rhizomucor miehei, from which mycelia and low molecular weight substances had been removed was subjected to anion exchange chromatography. The main lipolytic fraction from the column was desalted and ultrafiltrated prior to lyophilization. The lyophilized powder was then subjected to an affinity chromatography. The pooled lipase fractions from the column were desalted and concentrated by ultrafiltration. This concentrate was then subjected to a hydrophobic interaction chromatography (HIC) and the lipase from the HIC-purification was used for amino acid sequence determination. The sequence determination was carried out both on the native enzyme (N-terminal seqeunce) and on selected fragments obtained after proteolytic digestion of the lipase with Armillaria mellea protease. The sequence determination was performed with a Gas Phase Sequencer (Applied Biosystems Model 470A) as described by Thim, L. et al. (FEBS Lett. 1987, in press). RML was digested with Armillaria mellea protease as described by Moody et al. (FEBS Lett. 172 (1984), 142-148) with the only exception that the enzyme to substrate ration was 1:40 (mol:mol). Fragments obtained were separated by HPLC and the UV-absorption was monitored at 280 nm and 214 nm. In order to identify suitable fragments for the construction of oligonucleotide probes, only peptides which showed a high ratio between 280 nm and 214 nm were sequenced as these fragments contain Trp and/or Tyr.

The following N-terminal sequence was found by use of the native RML:

5                   10                   15
Ser-Ile-Asp-Gly-Gly-Ile-Arg-Ala-Ala-Thr-Ser-Gln-Glu-Ile-Asn-
20                   25
Glu-Leu-Thr-Tyr-Tyr-Thr-X-Leu-(Ser)-(Ala)-.

One of the fragments isolated from the proteolytic digest had the sequence of: Arg-Thr-Val-Ile-Pro-Gly-Ala-Thr-Trp-Asp-X-Ile-His, and this fragment was used for the synthesis of a specific oligonucleotide probe.

The Rhizomucor miehei cDNA library from example 3 constructed for isolation of the aspartic proteinase (RMP) recombinants from this organism was also used for identification of lipase specific recombinants. A mixture of oligonucleotides was synthesized on an Applied Biosystems Inc. DNA-synthesizer. The mixture which has the structure: 
   A
5'  TCCCANGTNGCNCC 3'  430/431
   G
 was complementary to RML mRNA in a region encoding the amino acids Gly-Ala-Thr-Trp-Asp. This pentapeptide was identified as a segment of an amino acid sequence obtained from proteolytic fragments of the purified RML protein (see above).

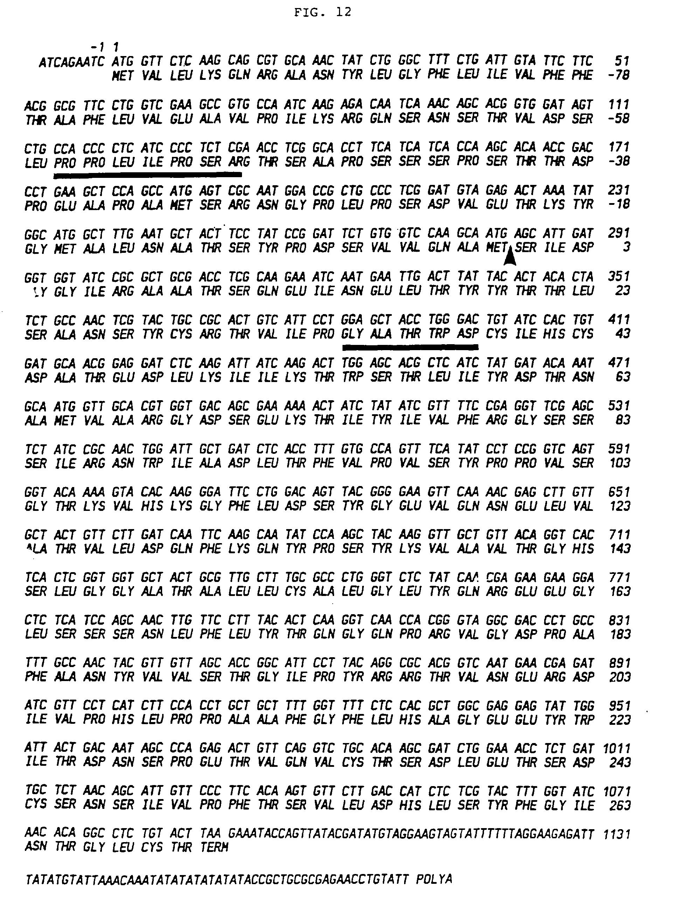

The Rhizomucor miehei cDNA library was screened with the ³²P-kinased lipase oligonucleotide mixture as described for screening with the RMP specific mixture. Hybridization and initial washing of the filters were done at 43°C. After autoradiography, the filters were washed at 47°C. Colonies that showed strong hybridization were isolated and the inserted cDNAs in the corresponding plasmids were sequenced to identify RML specific recombinants. Two such recombinants p353.7 and p353.16 had inserts of about 1.2 kb. The DNA sequence obtained from these two recombinants starts in the middle of the signal peptide (see Fig. 12) and extends through to the poly A tail. In this region one long open reading frame could be identified. Since the two recombinants did not include sequence for the 5' part of a signal peptide with its initiating methionine codon a synthetic oligonucleotide (584) 5' CGAGAGGGGATGAGGGGTGG 3' 584 was synthesized. This oligonucleotide 584 is complementary to the RML mRNA in a region encoding the amino acid sequence Pro-Pro-Leu-Ile-Pro-Ser-Arg found in the propeptide region (see fig. 12). After the oligo 584 had been kinased to high specific activity with T4 polynucleotide kinase and ³²P-γ-ATP, it was used in a primer extension reaction on Rhizomucor miehei mRNA with AMV reverse transcriptase according to published procedures (Boel, E. et al., PNAS, USA, 80, 2866-2869, 1983). The primer extension reaction products were electrophoresed on a 10% polyacrylamide/urea gel and two cDNA products were resolved. These two cDNAs, one 150 nucleotides long and the other 160 nucleotides long were both electroeluted and sequenced by the chemical degradation procedure for DNA sequencing. Both cDNAs gave readable sequence extending from the primer region and up to a position 9 nucleotides 5' to an initiating methionine codon. The sequence confirmed the sequence obtained from the lipase recombinant cDNA plasmids. The lengths of the two primer extension cDNA products indicate that the 5'end (CAP-site) of the lipase mRNA will be located app. 5 or 15 nucleotides 5' to the first A nucleotide shown in Fig. 12. A microheterogeniety in the location of the 5'end of mRNA's from fungi is very common. By combining the sequence obtained from the two cloned cDNAs p353.7 and p353.16 with the sequence from the primer extension analysis, the amino acid sequence of a RML precursor can be established. The DNA-sequence and the corresponding amino acid sequence of the RML precursor is shown in Fig. 12. In Fig. 12 the horizontal lines indicate the positions of synthetic oligos used for cDNA synthesis and for cDNA library screening. An arrow shows the position where processing occurs in maturation of native RML. Nucleotides are numbered from the first base in the initiating Met-codon and amino acids are numbered from the first residue in the mature native RML. The RML is encoded by an open reading frame extending from an initiating Met codon and then through 363 codons before a stop codon is reached. In this precursor the first 24 amino acid residues would constitute a typical hydrophobic signal peptide. According to the productive rules of von Heijne (Eur.J.Biochem. 133, 17 - 21, 1983), the signal peptide would be cleaved from the following propeptide by a signal peptidase cleavage between the Ala- and Val residues at position -71 and -70, respectively.

Since the N-terminal amino acid sequence analysis of purified RML obtained from the culture supernatant from Rhizomucor miehei identified Ser-Ile-Asp-Gly-Gly-Ile-Arg as the N-terminus of the active RML enzyme, the propeptide of the RML precursor consisted of the next 70 amino acid residues in the precursor. Beginning with this N-terminal Ser residue, the mature RML extends through 269 residues before reaching a termination codon. In this mature 29500 dalton enzyme a lipase substrate binding site is located around residue Ser(144) which is conserved in a number of lipases. In the 3' end of the RML mRNA 104 nucleotides were localized as an untranslated region between the TAA stop codon and the poly(A) tail. 23 nucleotides 5' to this poly(A) tail a repetitive structure consisting of 7 AT basepairs was found while no typical eukaryotic polyadenylation signal could be identified.

In a preferred embodiment of the present invention a number of changes was carried out on the RML cDNA. These changes involved removal of the G:C tails added to the cDNA during cloning and addition of restriction endonuclease sites 5' and 3' to the open reading frame. A number of convenient restriction sites were also introduced in the signal peptide and propeptide regions of the cDNA.

p353.16 was digested with FnuDII and a 880 bp DNA fragment (the 3'end of RML cDNA) was isolated by agarose gel electrophoresis. The fragment was electroeluted, phenol and CHCl₃ extracted and precipitated with ethanol.

This 3'end of RML cDNA was then ligated to a pUC19 vector that had been digested with SmaI and treated with alkaline phosphatase. The ligation reaction was used to transform competent E. coli cells and among the generated transformants correct recombinants were identified by restriction enzyme digestion of miniprep plasmids. One such appropriate recombinant p435.2 was digested with BanII and HindIII and a 0.69 kb fragment was isolated by agarose gel electrophoresis. The fragment was electroeluted, phenol and CHCl₃ extracted and precipitated with ethanol. This fragment of RML cDNA now had a major part of the pUC19 multiple cloning site joined to its 3' untranslated region.

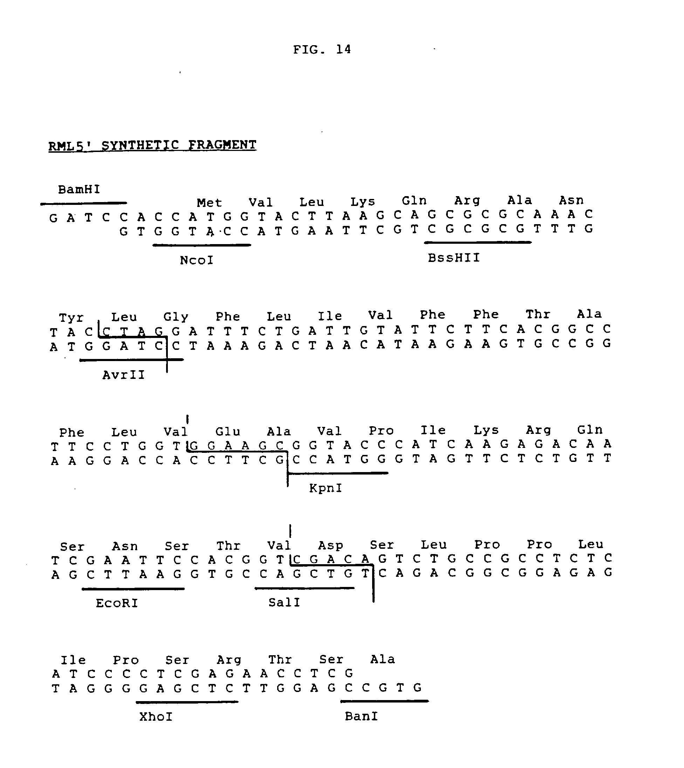

The 5' end of the RML cDNA was redesigned using synthetic oligonucleotides in order to introduce convenient. restriction sites. The DNA-sequence of the synthetic fragment (RML 5') is shown in Fig. 14. The position of introduced restriction sites and the joining sites of the individually synthesized oligonucleotides are indicated by horizontal viz. vertical/horizontal lines. The resulting fragment (RML 5') was purified as a 150 bp fragment on a 2% agarose gel, electroeluted, phenol and CHCl₃ extracted and precipitated with ethanol before further ligation reactions.

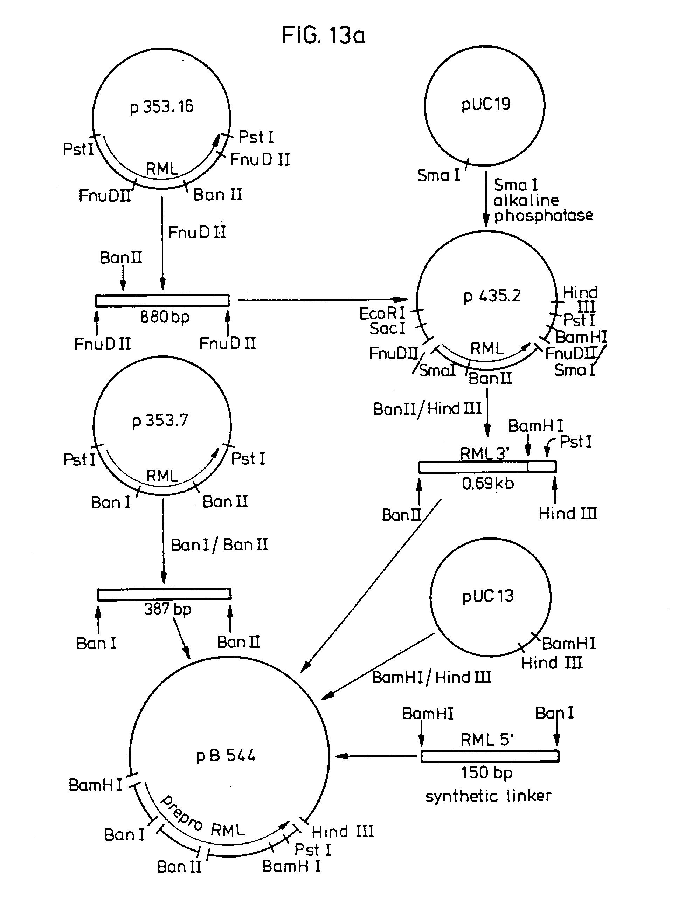

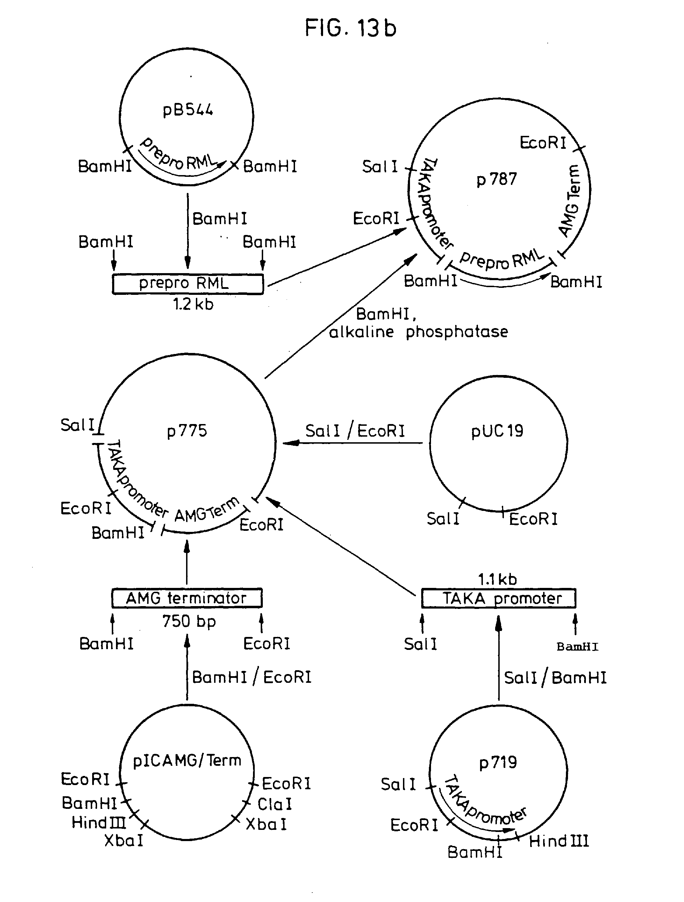

p353.7 was digested with BanI and BanII and a 387 bp RML fragment was purified by 10% polyacrylamide gel electrohporesis. The fragment was electroeluted, phenol and CHCl₃ extracted before ethanol precipitation and then ligated to the synthetic RML 5' fragment and the 0.69 kb BanII/HindIII fragment from p435.2 in a BamHI/HindIII digested pUC13 vector. The ligation reaction was used to transform competent E. coli cells and among the resulting transformants correct recombinants were identified by restriction enzyme digestions on miniprep plasmids. In one such correct recombinant, pB544, the synthetic part was sequenced to confirm the expected structure. The construction of pB544 is illustrated in Fig. 13a. From pB544 the prepro RML cDNA was isolated as a 1.2 kb BamHI fragment by agarose gel electrophoresis. An expression vector based on the promoter from the Aspergillus oryzae TAKA-amylase gene and the terminator from the Aspergillus niger glucoamylase gene was prepared as follows. p719 (see example 7) was digested with SalI and BamHI. The resulting 1.1 kb TAKA-amylase promoter fragment was purified by agarose gel electrophoresis. pICAMG/Term (see example 4) was digested with BamHI and EcoRI. The resulting 0.75 kb glucoamylase terminator fragment was purified by agarose gel electrophoresis. After phenol and CHCl₃ extractions these two fragments were ethanol precipitated and ligated to a SalI/EcoRI digested pUC19 vector. The ligation reaction was used to transform E. coli cells and among the resulting transformants correct recombinants were identified by restriction enzyme digestion of miniprep plasmids. One such correct recombinant p775 was digested with BamHI and treated with alkaline phosphatase. The 1.2 kb BamHI RML prepro cDNA fragment from pB544 was ligated to this p775 vector and transformed into E. coli. A recombinant p787 with the RML prepro cDNA inserted in the correct orientation between promoter and terminator was identified by restriction enzyme digestions on miniprep plasmids extracted from E. coli transformants. p787 plasmid DNA was grown in large scale and the plasmid preparation was purified by CsCl/Ethidium bromide centrifugation. The construction of p787 is illustrated in Fig. 13b.

### Example 9

### Transformation of Aspergillus oryzae (general procedure)

100 ml of YPD (Sherman et al., Methods in Yeast Genetics, Cold Spring Harbor Laboratory, 1981) was inoculated with spores of A. oryzae, IFO 4177 or argB mutants hereof and incubated with shaking at 37°C for about 2 days. The mycelium was harvested by filtration through miracloth and washed with 200 ml of 0.6 M MgSO₄. The mycelium was suspended in 15 ml of 1.2 M MgSO₄, 10 mM NaH₂PO₄, pH = 5.8. The suspension was cooled on ice and 1 ml of buffer containing 120 mg of Novozym® 234, batch 1687 was added. After 5 min., 1 ml of 12 mg/ml BSA (Sigma type H25) was added and incubation with gentle agitation continued for 1.5-2.5 hours at 37°C until a large number of protoplasts was visible in a sample inspected under the microscope.

The suspension was filtered through miracloth, the filtrate transferred to a sterile tube and overlayered with 5 ml of 0.6 M sorbitol, 100 mM Tris-HCl, pH = 7.0. Centrifugation was performed for 15 min. at 1000 g and the protoplasts were collected from the top of the MgSO₄ cushion. 2 volumes of STC (1.2 M sorbitol, 10 mM Tris-HCl pH = 7.5, 10 mM CaCl₂) were added to the protoplast suspension and the mixture was centrifugated for 5 min. at 1000 g. The protoplast pellet was resuspended in 3 ml of STC and repelleted. This was repeated. Finally the protoplasts were resuspended in 0.2-1 ml of STC.

100 µl of protoplast suspension was mixed with 5-25 µg of the appropriate DNA in 10 µl of STC. Protoplasts from the argB strains were mixed with pSal43 DNA (an A. nidulans argB gene carrying plasmid) and protoplasts from the argB⁺ strains were mixed with p3SR2 (an A. nidulans amdS gene carrying plasmid). The mixture was left at room temperature for 25 min. 0.2 ml of 60% PEG 4000 (BDH 29576), 10 mM CaCl₂ and 10 mM Tris-HCl pH = 7.5 was added and carefully mixed (twice) and finally 0.85 ml of the same solution was added and carefully mixed. The mixture was left at room temperature. for 25 min., spun at 2500 g for 15 min. and the pellet was resuspended in 2 ml of 1.2 M sorbitol. After one more sedimentation the protoplasts were spread on the appropriate plates. Protoplasts from the argB strains transformed with pSal43 were spread on minimal plates (Cove, Biochem.Biophys.Acta 113 (1966) 51-56) with glucose and urea as carbon and nitrogen sources respectively, and containing 1.2 M sorbitol for osmotic stabilization. Protoplasts from the argB⁺ strains transformed with p3SR2 were spread on minimal plates (Cove, Biochem.Biophys.Acta 113 (1966) 51-56) containing 1.0 M sucrose, pH = 7.0, 10 mM acetamide as nitrogen source and 20 mM CsCl to inhibit background growth. After incubation for 4-7 days at 37°C spores were picked, suspended in sterile water and spread for single colonies. This procedure was repeated and spores of a single colony after the second reisolation were stored as a defined transformant.

### Example 10

### Expression of TAKA-amylase in a wild type A. oryzae strain

pTAKAl7 was transformed into A. oryzae IFO 4177 by cotransformation with p3SR2 containing the amdS gene from A. nidulans as described in example 9. Protoplasts prepared as described were incubated with a mixture of equal amounts of pTAKA 17 and p3SR2, approximately 5 µg of each were used. 9 transformants which could use acetamide as sole nitrogen source were reisolated twice. After growth on YPD (Sherman et al, 1981) for three days culture supernatants were analysed by SDS-PAGE. The gels were stained with coomassie brilliant blue R. The best transformants produced 10 - 20 times more amylase than untransformed IFO 4177. One tranformant was selected for further studies and grown in a 2 liter Kieler fermentor on 4% soy bean meal and supplied with glucose during growth. The culture was heavily agitated during fermentation. Under these conditions IFO 4177 gave about 1 g/l and the transformant about 12 g/l of amylase determined as enzyme activity. Enzyme activity was measured as ability to degrade starch (Cereal Chemistry, 16 (1939), 712-723). The starch used was Merck Amylum solubile erg B.6 and the assay was performed at pH 4.7 and at 37°C. No external beta-amylase was added.

### Example 11

### Expression of RMP in A. oryzae

p777 from example 7 or p778 from example 6 was transformed into IFO-4177 by cotransformation with p3SR2 by the procedure described in example 9. Transformants were selected and reisolated as described in example 9.

Transformants were grown for three days in YPD and supernatants were analysed by SDS-PAGE followed by Western blotting and ELISA. The supernatants from transformants of both p777 and p778 contained from 50 - 150 mg/l of protein reacting with RMP antibody. The proteinase was overglycosylated compared to the R. miehei produced proteinase. Two forms were seen of which one is presumed to be a preform and the other the processed mature proteinase. Two transformants of p778 and three transformants of p777 were grown in a fermentor in the same way as the TAKA-amylase transformants described above. The two transformants of p778 gave approximately 0.2 g/l and 0.4 g/l and the three transformants of p777 gave approximately 0.5 g/l, 2.4 g/l and 3.3 g/l of RMP determined as milk clotting activity by the Kunitz method (Kunitz M., Jour.Gen.Physiol. 18 (1935), 459-466), assuming that the specific activity of the recombinant RMP is the same as that of the Rhizomucor miehei enzyme. (This has later been confirmed). SDS-PAGE and SDS-PAGE followed by Western-blotting and ELISA revealed that only one form of RMP was present when culturing in a larger scale. The RMP was overglycosylated also under these growth conditions. The protein amount seen on gels correlated well with the amounts predicted from enzyme activity.

RMP was purified from the culture supernatant by affinity chromatography and size exclusion chromatography.

The N-terminal sequence of the purified recombinant RMP was determined by use of a Gas Phase Sequencer as described by Thim et al. (FEBS Lett, 1987 in press).

Two forms of the recombinant RMP were found indicating that the processing in the N-terminal end was heterogeneous. One form had the N-terminal sequence of: Ala-Asp-Gly-Ser-Val-Asp-Thr-Pro-Gly-Tyr- and the other form had the N-terminal sequence of: Gly-Ser-Val-Asp-Thr-Pro-Gly-Tyr-Tyr-Asp-. Such heterogeneous processing at the N-terminus has also been described for native RMP from Mucor miehei (Paquet, D. et al., Neth.Milk.Dairy J., 35 (1981), 358-360). As the heterogenous processing of the recombinant RMP correlates well with that of native RMP, A. oryzae has according to the present invention been shown to be able to process recombinant RMP in the correct region.

### Example 12

### Construction of an Expression unit for the production of prochymosin in A. oryzae

The construction contains the prochymosin gene immediately preceded by the signal peptide sequence from the A. oryzae TAKA-amylase gene under control of the A. oryzae TAKA-amylase promoter. The construct further contains the terminator from the A. niger glucoamylase gene plus an E. coli replicon.

An approximately 430 bp BamHI/XmaI fragment from p285' proC (see example 1) and a synthetic oligomer of the following sequence 
AATTCCAGCTGCCGCGGCCGAGATCACCAG
GGTCGACGGCGCCGGCTCTAGTGGTCCTAG
 were inserted into EcoRI-XmaI cut pUC19 plasmid giving plasmid pToC50a.

pToC50a was cut with EcoRI-SacII and the large 0 fragment containing pUC19 and the 5' part of the prochymosin gene (prochymosin') was isolated. This fragment was ligated with a 0.6 kb EcoRI-BanI fragment from pTAKA 17 and the following synthetic oligomer

GCACCTGCTTTGGC
    GACGAAAC        (KFN 280/281)

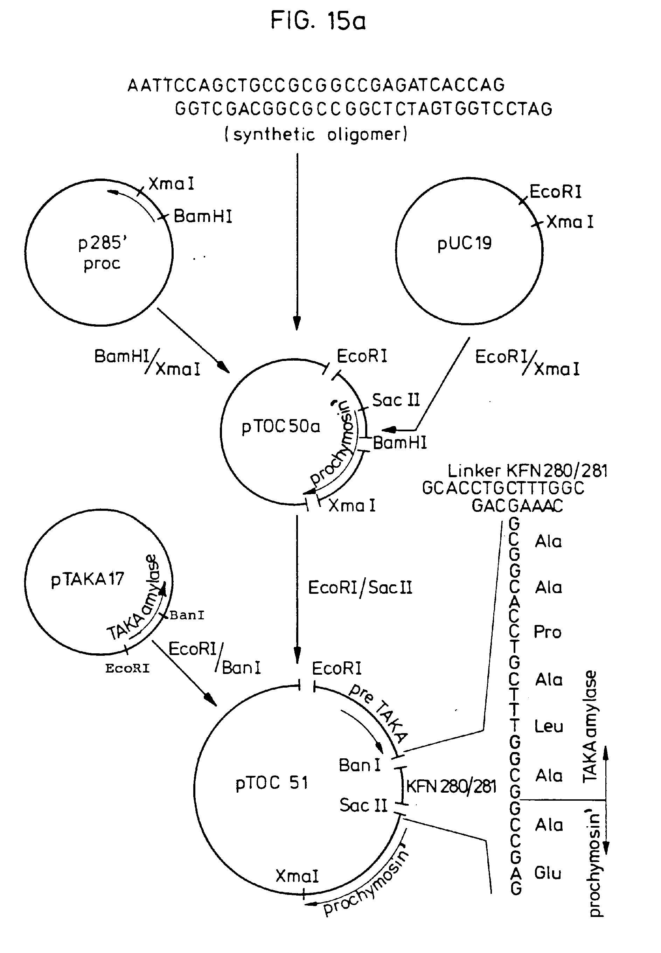

After transformation a plasmid pToC51 containing the 5' part of the prochymosin gene (prochymosin') fused to the signal sequence from the A. oryzae TAKA-amylase gene (preTAKA) and preceded by approximately 500 bp upstream TAKA-amylase sequence was isolated. The construction of pToC51 is illustrated in Fig. 15a.

pR26 was cut with HinfI, treated with the large fragment (Klenow) of DNA polymerase I and the four dNTP's and cut with XmaI. A 750 bp fragment containing the 3'end of the prochymosin gene was isolated. With the purpose of inserting a HindIII at the 3'end of this fragment pUC9 was cut with XmaI/HincII and the large fragment was ligated to the 750 bp fragment containing the 3'end of the prochymosin gene.

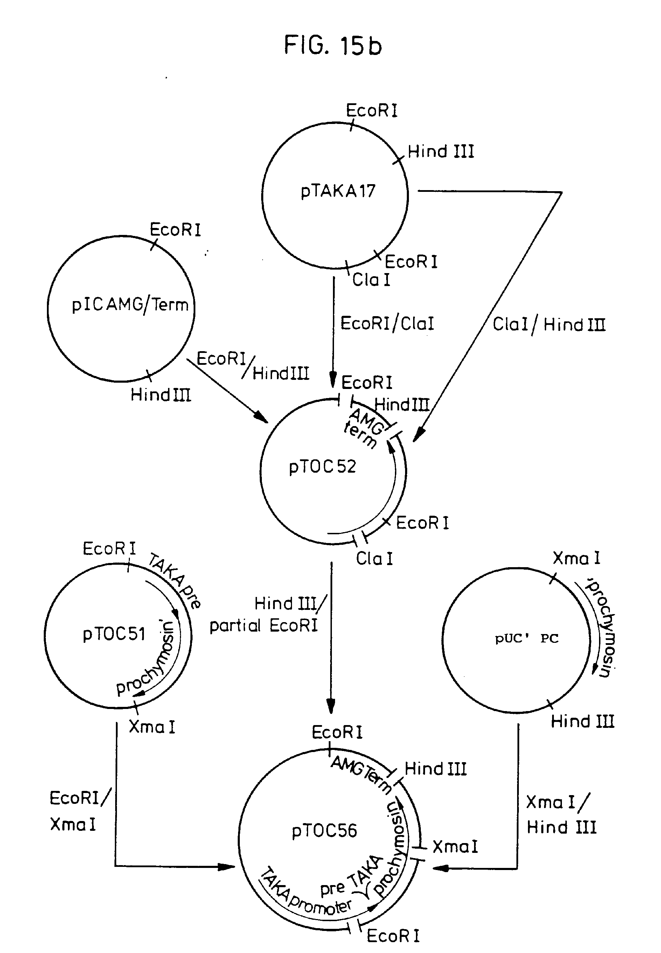

A 5.6 kb EcoRI-ClaI fragment from pTAKA 17 was isolated and ligated with a 2.6 kb ClaI-HindIII fragment from the same plasmid plus a 0.7 kb EcoRI-HindIII fragment from pICAMG/Term (see example 4) containing the A. niger glucoamylase gene terminator and polyA site. The resulting plasmid is illustrated in Fig. 15b as pToC52.

pToC 52 was cut with HindIII and partially with EcoRI and a 6.4 kb fragment was isolated. This was ligated with a 0.9 kb EcoRI-XmaI fragment from pToC51 and a 0.7 kb XmaI-HindIII fragment from pUC9'PC containing the 3' part of the prochymosin gene ('prochymosin). The resulting plasmid is called pToC56 and is depicted in Fig. 15b.

### Example 13

### Expression of prochymosin in A. oryzae

pToC56 was transformed into A. oryzae IFO 4177 or an argB mutant thereof by cotransformation with either p3SR2 (amdS gene) or pSal43 (argB gene). Transformants which grew on selective media were reisolated twice as described in example 9.

The transformants were grown for three days in YPD and the prochymosin content in supernatants was analysed by ELISA on a Western blot after SDS-PAGE. The transformants produced 1 - 10 mg/l of a prochymosin size immunoreactive protein in the supernatants. No other immunoreactive proteins were detected in the supernatants.

### Example 14

### Expression of RML in A. oryzae

p787 from example 8 was transformed into IFO-4177 by cotransformation with p3SR2 by the procedure described in example 9. Transformants were selected and reisolated as described in example 9.

Supernatants from YPD cultures of the transformants grown for three days were analyzed by SDS-PAGE followed by Western blotting and ELISA. The best transformant produced 2 mg/l of a protein the size of the matured RML. The lipase activity in the supernatants was assayed as the ability to cleave tributyrin (NOVO method AF 95.1/3-GB).

The measurement confirmed that 2 mg/l of active lipase was present in the supernatants.

## Claims

1. A process for expression of a protein product in Aspergillus oryzae comprising the steps of:
(a) providing a recombinant DNA cloning vector system capable of integration into the genome of an Aspergillus oryzae host in one or more copies and comprising: DNA-sequences encoding functions facilitating gene expression; a suitable marker for selection of transformants; and a DNA-sequence encoding the desired protein product;
(b) transforming the Aspergillus oryzae host which does not harbour a functional gene for the chosen selection marker with the recombinant DNA cloning vector system from step a; and
(c) culturing the transformed Aspergillus oryzae host in a suitable culture medium.

2. A process according to claim 1, wherein the DNA-sequences encoding functions facilitating gene expression comprises a promoter, transcription initiation sites, and transcription terminator and polyadenylation functions.

3. A process according to claim 2, wherein the promoter is preceded by upstream activating sequences.

4. A process according to claim 1, wherein the selection marker is derived from the gene for A. nidulans or A. niger argB, A. nidulans trpC, A. nidulans amdS, Neurospora crassae Pyr4 or DHFR.

5. A process according to claim 4, wherein the selection marker is the ArgB gene derived from A. nidulans or A. niger or the amdS gene derived from A. nidulans.

6. A process according claim 3, wherein the promoter and upstream activating sequences are derived from a gene encoding an extracellular or intracellular protein, such as an amylase, a glucoamylase, a protease, a lipase, a cellulase or a glycolytic enzyme.

7. A process according to claim 6, wherein the promoter and upstream activating sequences are derived from the gene for A. oryzae TAKA amylase, Rhizomucor miehei aspartic proteinase, A. niger neutral α-amylase, A. niger acid stable α-amylase, A. niger glucoamylase or Rhizomucor miehei lipase.

8. A process according to claim 7, wherein the promoter is the A. oryzae TAKA amylase promoter or functional parts thereof.

9. A process according to claim 8, wherein the promoter and upstream activating sequences have the following sequence: 
GTCGACGC ATTCCGAATA CGAGGCCTGA TTAATGATTA CATACGCCTC
CGGGTAGTAG ACCGAGCAGC CGAGCCAGTT CAGCGCCTAA AACGCCTTAT
ACAATTAAGC AGTTAAAGAA GTTAGAATCT ACGCTTAAAA AGCTACTTAA
AAATCGATCT CGCAGTCCCG ATTCGCCTAT CAAAACCAGT TTAAATCAAC
TGATTAAAGG TGCCGAACGA GCTATAAATG ATATAACAAT ATTAAAGCAT
TAATTAGAGC AATATCAGGC CGCGCACGAA AGGCAACTTA AAAAGCGAAA
GCGCTCTACT AAACAGATTA CTTTTGAAAA AGGCACATCA GTATTTAAAG
CCCGAATCCT TATTAAGCGC CGAAATCAGG CAGATAAAGC CATACAGGCA
GATAGACCTC TACCTATTAA ATCGGCTTCT AGGCGCGCTC CATCTAAATG
TTCTGGCTGT GGTGTACAGG GGCATAAAAT TACGCACTAC CCGAATCGAT
AGAACTACTC ATTTTTATAT AGAAGTCAGA ATTCATAGTG TTTTGATCAT
TTTAAATTTT TATATGGCGG GTGGTGGGCA ACTCGCTTGC GCGGGCAACT
 
CGCTTACCGA TTACGTTAGG GCTGATATTT ACGTGAAAAT CGTCAAGGGA
TGCAAGACCA AAGTAGTAAA ACCCCGGAAG TCAACAGCAT CCAAGCCCAA
GTCCTTCACG GAGAAACCCC AGCGTCCACA TCACGAGCGA AGGACCACCT
CTAGGCATCG GACGCACCAT CCAATTAGAA GCAGCAAAGC GAAACAGCCC
AAGAAAAAGG TCGGCCCGTC GGCCTTTTCT GCAACGCTGA TCACGGGCAG
CGATCCAACC AACACCCTCC AGAGTGACTA GGGGCGGAAA TTTAAAGGGA
TTAATTTCCA CTCAACCACA AATCACAGTC GTCCCCGGTA TTGTCCTGCA
GAATGCAATT TAAACTCTTC TGCGAATCGC TTGGATTCCC CGCCCCTAGT
CGTAGAGCTT AAAGTATGTC CCTTGTCGAT GCGATGTATC ACAACATATA
AATACTAGCA AGGGATGCCA TGCTTGGAGG ATAGCAACCG ACAACATCAC
ATCAAGCTCT CCCTTCTCTG AACAATAAAC CCCACAG
 or a functionally equivalent nucleotide sequence.

10. A process according to claim 8, wherein the promoter and upstream activating sequences have the following sequence: 
AGATCTGCCC TTATAAATCT CCTAGTCTGA TCGTCGACGC ATTCCGAATA
CGAGGCCTGA TTAATGATTA CATACGCCTC CGGGTAGTAG ACCGAGCAGC
CGAGCCAGTT CAGCGCCTAA AACGCCTTAT ACAATTAAGC AGTTAAAGAA
GTTAGAATCT ACGCTTAAAA AGCTACTTAA AAATCGATCT CGCAGTCCCG
ATTCGCCTAT CAAAACCAGT TTAAATCAAC TGATTAAAGG TGCCGAACGA
GCTATAAATG ATATAACAAT ATTAAAGCAT TAATTAGAGC AATATCAGGC
CGCGCACGAA AGGCAACTTA AAAAGCGAAA GCGCTCTACT AAACAGATTA
CTTTTGAAAA AGGCACATCA GTATTTAAAG CCCGAATCCT TATTAAGCGC
CGAAATCAGG CAGATAAAGC CATACAGGCA GATAGACCTC TACCTATTAA
ATCGGCTTCT AGGCGCGCTC CATCTAAATG TTCTGGCTGT GGTGTACAGG
GGCATAAAAT TACGCACTAC CCGAATCGAT AGAACTACTC ATTTTTATAT
AGAAGTCAGA ATTCATAGTG TTTTGATCAT TTTAAATTTT TATATGGCGG
GTGGTGGGCA ACTCGCTTGC GCGGGCAACT CGCTTACCGA TTACGTTAGG
GCTGATATTT ACGTGAAAAT CGTCAAGGGA TGCAAGACCA AAGTAGTAAA
ACCCCGGAAG TCAACAGCAT CCAAGCCCAA GTCCTTCACG GAGAAACCCC
AGCGTCCACA TCACGAGCGA AGGACCACCT CTAGGCATCG GACGCACCAT
CCAATTAGAA GCAGCAAAGC GAAACAGCCC AAGAAAAAGG TCGGCCCGTC
GGCCTTTTCT GCAACGCTGA TCACGGGCAG CGATCCAACC AACACCCTCC
AGAGTGACTA GGGGCGGAAA TTTAAAGGGA TTAATTTCCA CTCAACCACA
 
AATCACAGTC GTCCCCGGTA TTGTCCTGCA GAATGCAATT TAAACTCTTC
TGCGAATCGC TTGGATTCCC CGCCCCTAGT CGTAGAGCTT AAAGTATGTC
CCTTGTCGAT GCGATGTATC ACAACATATA AATACTAGCA AGGGATGCCA
TGCTTGGAGG ATAGCAACCG ACAACATCAC ATCAAGCTCT CCCTTCTCTG
AACAATAAAC CCCACAGAAG GCATTT
 or a functionally equivalent nucleotide sequence.

11. A process according to claim 10, wherein the sequence in claim 10 is preceded by the 1.05 kb unsequenced upstream region from position 0 to 1.05 in plasmid pTAKA 17.

12. A process according to claim 1, wherein the vector system further comprises a preregion providing for secretion of the expressed product into the culture medium.

13. A process according to claim 12, wherein the preregion is derived from a glucoamylase or an amylase gene from an Aspergillus species, an amylase gene from a Bacillus species, a lipase or proteinase gene from Rhizomucor miehei, the gene for the α-factor from S. cerevisiae or the calf prochymosin gene.

14. A process according to claim 13, wherein the preregion is derived from the gene for A. oryzae TAKA amylase, A. niger neutral α-amylase, A. niger acid-stable α-amylase, B. licheniformis α-amylase, the maltogenic amylase from Bacillus NCIB 11837, B. stearothermophilus α-amylase or B. licheniformis subtilisin.

15. A process according to claim 14, wherein the preregion is the TAKA-amylase preregion with the following sequence

ATGATGGTCGCGTGGTGGTCTCTATTTCTGTACGGCCTTCAGGTCGCGGCACCT
MetMetValAlaTrpTrpSerLeuPheLeuTyrGlyLeuGlnValAlaAlaPro

GCTTTGGCT
AlaLeuAla

16. A process according to claim 1, wherein the vector system comprises two vectors, where one contains the selection marker and the other contains DNA-sequences encoding functions facilitating gene expression and a DNA-sequence encoding the desired protein product.

17. A process for production of a protein product in Aspergillus oryzae, wherein an Aspergillus oryzae strain being transformed with a recombinant DNA cloning vector system as described in claim 1 is cultured in a suitable culture medium and the product is recovered from the culture medium.

18. A promoter suitable for expression of a protein product in Aspergillus, **characterized by** being the TAKA-amylase promoter or functional parts thereof optionally preceded by upstream activating sequences.

19. A promcter and upstream activating sequences according to claim 18 having the following sequence 
AGATCTGCCC TTATAAATCT CCTAGTCTGA TCGTCGACGC ATTCCGAATA
CGAGGCCTGA TTAATGATTA CATACGCCTC CGGGTAGTAG ACCGAGCAGC
CGAGCCAGTT CAGCGCCTAA AACGCCTTAT ACAATTAAGC AGTTAAAGAA
GTTAGAATCT ACGCTTAAAA AGCTACTTAA AAATCGATCT CGCAGTCCCG
ATTCGCCTAT CAAAACCAGT TTAAATCAAC TGATTAAAGG TGCCGAACGA
GCTATAAATG ATATAACAAT ATTAAAGCAT TAATTAGAGC AATATCAGGC
CGCGCACGAA AGGCAACTTA AAAAGCGAAA GCGCTCTACT AAACAGATTA
CTTTTGAAAA AGGCACATCA GTATTTAAAG CCCGAATCCT TATTAAGCGC
CGAAATCAGG CAGATAAAGC CATACAGGCA GATAGACCTC TACCTATTAA
ATCGGCTTCT AGGCGCGCTC CATCTAAATG TTCTGGCTGT GGTGTACAGG
 
GGCATAAAAT TACGCACTAC CCGAATCGAT AGAACTACTC ATTTTTATAT
AGAAGTCAGA ATTCATAGTG TTTTGATCAT TTTAAATTTT TATATGGCGG
GTGGTGGGCA ACTCGCTTGC GCGGGCAACT CGCTTACCGA TTACGTTAGG
GCTGATATTT ACGTGAAAAT CGTCAAGGGA TGCAAGACCA AAGTAGTAAA
ACCCCGGAAG TCAACAGCAT CCAAGCCCAA GTCCTTCACG GAGAAACCCC
AGCGTCCACA TCACGAGCGA AGGACCACCT CTAGGCATCG GACGCACCAT
CCAATTAGAA GCAGCAAAGC GAAACAGCCC AAGAAAAAGG TCGGCCCGTC
GGCCTTTTCT GCAACGCTGA TCACGGGCAG CGATCCAACC AACACCCTCC
AGAGTGACTA GGGGCGGAAA TTTAAAGGGA TTAATTTCCA CTCAACCACA
AATCACAGTC GTCCCCGGTA TTGTCCTGCA GAATGCAATT TAAACTCTTC
TGCGAATCGC TTGGATTCCC CGCCCCTAGT CGTAGAGCTT AAAGTATGTC
CCTTGTCGAT GCGATGTATC ACAACATATA AATACTAGCA AGGGATGCCA
TGCTTGGAGG ATAGCAACCG ACAACATCAC ATCAAGCTCT CCCTTCTCTG
AACAATAAAC CCCACAGAAG GCATTT
 or a functionally equivalent nucleotide sequence.

20. A promoter and upstream activating sequences according to claim 19, wherein the upstream activating sequences are preceded by the 1.05 kb unsequenced upstream region from position 0 to 1.05 in plasmid pTAKA 17.

21. A promoter and upstream activating sequences according to claim 19 having the following sequence 
GTCGACGC ATTCCGAATA CGAGGCCTGA TTAATGATTA CATACGCCTC
CGGGTAGTAG ACCGAGCAGC CGAGCCAGTT CAGCGCCTAA AACGCCTTAT
ACAATTAAGC AGTTAAAGAA GTTAGAATCT ACGCTTAAAA AGCTACTTAA
AAATCGATCT CGCAGTCCCG ATTCGCCTAT CAAAACCAGT TTAAATCAAC
TGATTAAAGG TGCCGAACGA GCTATAAATG ATATAACAAT ATTAAAGCAT
TAATTAGAGC AATATCAGGC CGCGCACGAA AGGCAACTTA AAAAGCGAAA
GCGCTCTACT AAACAGATTA CTTTTGAAAA AGGCACATCA GTATTTAAAG
CCCGAATCCT TATTAAGCGC CGAAATCAGG CAGATAAAGC CATACAGGCA
GATAGACCTC TACCTATTAA ATCGGCTTCT AGGCGCGCTC CATCTAAATG
TTCTGGCTGT GGTGTACAGG GGCATAAAAT TACGCACTAC CCGAATCGAT
AGAACTACTC ATTTTTATAT AGAAGTCAGA ATTCATAGTG TTTTGATCAT
TTTAAATTTT TATATGGCGG GTGGTGGGCA ACTCGCTTGC GCGGGCAACT
CGCTTACCGA TTACGTTAGG GCTGATATTT ACGTGAAAAT CGTCAAGGGA
 
TGCAAGACCA AAGTAGTAAA ACCCCGGAAG TCAACAGCAT CCAAGCCCAA
GTCCTTCACG GAGAAACCCC AGCGTCCACA TCACGAGCGA AGGACCACCT
CTAGGCATCG GACGCACCAT CCAATTAGAA GCAGCAAAGC GAAACAGCCC
AAGAAAAAGG TCGGCCCGTC GGCCTTTTCT GCAACGCTGA TCACGGGCAG
CGATCCAACC AACACCCTCC AGAGTGACTA GGGGCGGAAA TTTAAAGGGA
TTAATTTCCA CTCAACCACA AATCACAGTC GTCCCCGGTA TTGTCCTGCA
GAATGCAATT TAAACTCTTC TGCGAATCGC TTGGATTCCC CGCCCCTAGT
CGTAGAGCTT AAAGTATGTC CCTTGTCGAT GCGATGTATC ACAACATATA
AATACTAGCA AGGGATGCCA TGCTTGGAGG ATAGCAACCG ACAACATCAC
ATCAAGCTCT CCCTTCTCTG AACAATAAAC CCCACAG
 or a functionally equivalent nucleotide sequence.
